# EUROPEAN PATENT SPECIFICATION

(11) **EP 2 739 972 B1**
(45) Date of publication and mention of the grant of the patent: **04.07.2018**
(21) Application number: 12821914.4
(22) Date of filing: 05.08.2012
(51) Int. Cl.: G01N 33/50

(54) **METHOD OF IDENTIFYING AGENTS THAT AFFECT MATURATION, SURVIVAL AND MYELINATION**
VERFAHREN ZUR IDENTIFIZIERUNG VON WIRKSTOFFEN FÜR REIFUNG, ÜBERLEBEN UND MYELINISIERUNG
PROCÉDÉ D'IDENTIFICATION D'AGENTS QUI AFFECTENT LA MATURATION, LA SURVIE ET LA MYÉLINATION

(30) Priority: 07.08.2011 US 201161515926 P
(43) Date of publication of application: 11.06.2014
(73) Proprietor: Kadimastem Ltd., 74140 Nes-Ziona (IL)
(72) Inventor: REVEL, Michel, Rehovot (IL); CHEBATH, Judith, Rehovot (IL); HASSON, Arye, 55401 Kiryat Ono (IL); IZRAEL, Michal, Rehovot (IL); KAUFMAN, Rosalia, Rehovot (IL)
(74) Representative: Becker Kurig Straus
(86) International application number: PCT/IL2012/050289
(87) International publication number: WO 2013/021381

(56) References cited:
- WO-A1-2004/111210
- WO-A2-2008/026198
- US-A1- 2006 172 415
- US-A1- 2007 231 898
- US-A1- 2009 196 859
- CUI QIAO LING ET AL: "Response of human oligodendrocyte progenitors to growth factors and axon signals", JOURNAL OF NEUROPATHOLOGY AND EXPERIMENTAL NEUROLOGY, USA, vol. 69, no. 9, 1 September 2010 (2010-09-01), pages 930-944, XP009182397, ISSN: 1554-6578, DOI: 10.1097/NEN.0B013E3181EF3BE4
- IZRAEL ET AL.: 'Human oligodendrocytes derived from embryonic stem cells: Effect of noggin on phenotypic differentiation in vitro and on myelination in vivo.' MOL CELL NEUROSCI vol. 34, no. 3, 28 December 2006, pages 310 - 323, XP005907285
- ZHANG ET AL.: 'Increased myelinating capacity of embryonic stem cell derived oligodendrocyte precursors after treatment by interleukin-6/soluble interleukin-6 receptor fusion protein.' MOL CELL NEUROSCI vol. 31, no. 3, 01 December 2005, pages 387 - 298, XP024908207
- GIL ET AL.: 'Vitronectin promotes oligodendrocyte differentiation during neurogenesis of human embryonic stem cells.' FEBS LETTERS vol. 583, 19 January 2009, pages 561 - 567, XP025923241

### SEQUENCE LISTING

<110> Kadimastem Ltd. Chebath, Judith Izrael, Michal Kaufman, Rosalia Hasson, Arik Revel, Michel
<120> METHOD OF IDENTIFYING AGENTS THAT STIMULATE MYELINATION
<130> 51573
<160> 20
<170> PatentIn version 3.5
<210> 1
   <211> 22
   <212> DNA
   <213> Artificial sequence
<220>
   <223> Single strand DNA oligonucleotide
<400> 1
   tgcctctcct tctgaacctt gg 22
<210> 2
   <211> 20
   <212> DNA
   <213> Artificial sequence
<220>
   <223> Single strand DNA oligonucleotide
<400> 2
   gcgaaatctg ccaccagttg 20
<210> 3
   <211> 24
   <212> DNA
   <213> Artificial sequence
<220>
   <223> Single strand DNA oligonucleotide
<400> 3
   catgtaggcc atgaggtcca ccac 24
<210> 4
   <211> 26
   <212> DNA
   <213> Artificial sequence
<220>
   <223> Single strand DNA oligonucleotide
<400> 4
   tgaaggtcgg tgtgaacgga tttggc 26
<210> 5
   <211> 19
   <212> DNA
   <213> Artificial sequence
<220>
   <223> Single strand DNA oligonucleotide
<400> 5
   tggccaagcc agacctcac 19
<210> 6
   <211> 21
   <212> DNA
   <213> Artificial sequence
<220>
   <223> Single strand DNA oligonucleotide
<400> 6
   catctccacg gtcttcacca c 21
<210> 7
   <211> 18
   <212> DNA
   <213> Artificial sequence
<220>
   <223> Single strand DNA oligonucleotide
<400> 7
   cagttcctga cgctccac 18
<210> 8
   <211> 23
   <212> DNA
   <213> Artificial sequence
<220>
   <223> Single strand DNA oligonucleotide
<400> 8
   cggcactaca aatatcatag aac 23
<210> 9
   <211> 24
   <212> DNA
   <213> Artificial sequence
<220>
   <223> Single strand DNA oligonucleotide
<400> 9
   ttgcatccag tgttcccgat ttac 24
<210> 10
   <211> 24
   <212> DNA
   <213> Artificial sequence
<220>
   <223> Single strand DNA oligonucleotide
<400> 10
   tgccagttaa attcggctac tacc 24
<210> 11
   <211> 22
   <212> DNA
   <213> Artificial sequence
<220>
   <223> Single strand DNA oligonucleotide
<400> 11
   aaggaggcag tggcttcaag tc 22
<210> 12
   <211> 20
   <212> DNA
   <213> Artificial sequence
<220>
   <223> Single strand DNA oligonucleotide
<400> 12
   cgctcaccag tcgcttcatc 20
<210> 13
   <211> 20
   <212> DNA
   <213> Artificial sequence
<220>
   <223> Single strand DNA oligonucleotide
<400> 13
   ctgctcacct tcatgattgc 20
<210> 14
   <211> 20
   <212> DNA
   <213> Artificial sequence
<220>
   <223> Single strand DNA oligonucleotide
<400> 14
   tgacttgcag ttgggaagtc 20
<210> 15
   <211> 21
   <212> DNA
   <213> Artificial sequence
<220>
   <223> Single strand DNA oligonucleotide
<400> 15
   gtatgtcatc tatggaactg c 21
<210> 16
   <211> 20
   <212> DNA
   <213> Artificial sequence
<220>
   <223> Single strand DNA oligonucleotide
<400> 16
   gtaatgtaca caggcacagc 20
<210> 17
   <211> 25
   <212> DNA
   <213> Artificial sequence
<220>
   <223> Single strand DNA oligonucleotide
<400> 17
   gcctgttctc ctggggcttt gctgc 25
<210> 18
   <211> 25
   <212> DNA
   <213> Artificial sequence
<220>
   <223> Single strand DNA oligonucleotide
<400> 18
   catccacctc acagatcgcc tacac 25
<210> 19
   <211> 21
   <212> DNA
   <213> Artificial sequence
<220>
   <223> Single strand DNA oligonucleotide
<400> 19
   ccatggctag acgctgaaaa c 21
<210> 20
   <211> 21
   <212> DNA
   <213> Artificial sequence
<220>
   <223> Single strand DNA oligonucleotide
<400> 20
   gaaggaacgt gatcttcaca c 21

## Description

### FIELD AND BACKGROUND OF THE INVENTION

The invention, in some embodiments thereof, relates to a method of identifying agents that affect maturation and survival of oligodendrocytes or myelination of neuronal cells using *ex-vivo* differentiated embryonic stem cells.

Myelin, the fatty substance, which encloses certain axons and nerve fibers, provides essential insulation, and enables the conductivity of nerve cells, which transmit electrical messages to and from the brain.

Aberration in myelination can lead to several pathologies in the central nervous system. These include, for example, autoimmune diseases (e.g multiple sclerosis), congenital leukodystrophies (e.g Pelizaeus-Merzbacher, vanishing white matter, adrenoleukodystrophy), infectious diseases (e.g. progressive multifocal leukoencephalopathy, postinflammatory demyelinated lesions), neurodegenerative diseases (e.g. multisystem degeneration), vascular diseases (vascular leukoencephalopathies, subcortical infarcts), neurodegenerative diseases (e.g. amyotrophic lateral sclerosis [ALS], Alzheimer's disease, Parkinson's disease), demyelinative brain or spinal cord trauma or injuries, and possibly neoplasms (e.g. oligodendrio-glioma).

Treatment of these pathologies may be effected by cell replacement therapies, which eventually may allow achieving regional or even global repair of myelin as indicated by experiments in MBP-deficient shiverer mice (e.g.Yandava et al., 1999, Proc Natl Acad Sci USA 96, 7029-34).

Oligodendrocytes extend as many as 50 processes, which wrap around axons to form myelin sheaths. *In-vitro* and *in-vivo,* the development of oligodendrocytes proceeds in steps from neural stem cells to bipolar progenitors, then to multipolar precursor cells having several main processes which subsequently arborize and form the multiple branches of the mature cells (Rogister et al., 1999, Mol Cell Neurosci 14, 287-300; Grinspan, 2002, J Neuropathol Exp Neurol 61, 297-306). Early oligodendrocytes precursors (OP) express the PDGF-receptor and later the specific O4 sulfatide marker of pre-oligodendrocytes which persists in ramified immature oligodendrocytes, then the maturating cells express galactocerebrosides (O1, GalC) and 2',3' cyclic nucleotide 3' phosphodiesterase (CNP), and at the final stage become postmitotic mature oligodendrocytes that synthesize the myelin membrane components such as proteolipid protein (PLP) and myelin basic protein (MBP).

Until presently, mechanistic studies analyzing human oligodendrocyte differentiation and functional myelination have been based on rodent assays. However the significant differences in gene expression and dominant signaling pathways between rodent and human's oligodendrocyte progenitor cells (OPCs) suggest that such assays may provide false results [Sim, F.J., et al., Neuron Glia Biol 5, 45-55 (2009)].

A co-culture system using brain derived OPCs from human embryos was recently described [Cui, Q.L. et al. Response of human oligodendrocyte progenitors to growth factors and axon signals. J Neuropathol Exp Neurol 69, 930-944]. However, the use of human embryos is problematic, due to lack of reproducibility and poor availability.

U.S. Patent Application No. 20100003751 teaches generation of oligodendrocytes from human embryonic stem cells.

US 2006/0172415 discloses a method of screening for compounds that affect a biological function of OPCs, said method comprising contacting a self-renewing, phenotypically homogenous population of OPCs with a test compound, and detecting an increase or decrease of myelination. Said document does not disclose a specific expression pattern of said OPCs including expression of oligl, olig2 and Hes5.

### SUMMARY OF THE INVENTION

According to one aspect of the present invention, there is provided a method of quantifying an effect of an agent on myelination of neuronal cells, the method comprising:
(a) *ex-vivo* differentiating embryonic stem cells into OPCs expressing Hes5, oligl and olig2;
(b) freezing and thawing said OPCs, wherein thawing is performed in a medium comprising growth factors;
(c) after thawing, culturing said OPCs for at least one day in the absence of growth factors;
(d) contacting said OPCs with the agent; and
(e) quantifying at least one marker of myelination in the OPCs, thereby quantifying the effect of the agent on myelination of neuronal cells.

According to another aspect of the present invention, there is provided a method of quantifying an effect of an agent on maturation, survival or a combination thereof of oligodendrocytes, the method comprising:
(a) *ex-vivo* differentiating embryonic stem cells into OPCs expressing Hes5, oligl and olig2;
(b) freezing and thawing said OPCs, wherein thawing is performed in a medium comprising growth factors;
(c) after thawing, culturing said OPCs for at least one day in the absence of growth factors;
(d) contacting said OPCs with the agent; and
(e) quantifying at least one marker of maturation, survival or a combination thereof in the oligodendrocytes, thereby quantifying the effect of the agent on maturation, survival or a combination thereof of the oligodendrocytes.

According to some embodiments of the invention, the OPCs express oligl and olig2 to a greater extent than they express myelin basic protein (MBP).

According to some embodiments of the invention, the marker is a polypeptide selected from the group consisting of myelin basic protein (MBP), myelin oligodendrocyte glycoprotein (MOG), galactocerebroside (GalC), myelin associated glycoprotein (MAG), 2'3'-cyclic nucleotide-30-phosphodiesterase (CNP), O4 and proteolipid protein (PLP).

According to some embodiments of the invention, the quantification of the effect of the agent on myelination, maturation, survival, or a combination thereof is conducted by counting the number of O4 stained cells with at least two processes, the percentage of O4 positive cells, the number of processes per O4 stained cells, the total length of the processes per O4 stained cells, the number of branch points per O4 stained cells, the number of MBP positive cells, calculating the percentage of MBP positive cells or measuring the area of myelin membranes per stained cells or the area of the O4 stained processes.

According to some embodiments of the invention, the agent is a small molecule.

According to some embodiments of the invention, the OPCs are present in a culture comprising neuronal cells.

According to some embodiments of the invention, the neuronal cells are human neuronal cells.

According to some embodiments of the invention, the neuronal cells are rodent neuronal cells.

According to some embodiments of the invention, the culture is devoid of Schwann cells. According to some embodiments of the invention, the Schwann cells are eliminated by 5'-fluoro-2'-deoxyuridine (FUdR)

According to some embodiments of the invention, the method further comprises quantifying an amount of myelination of neuronal cells in the culture comprising neuronal cells.

According to some embodiments of the invention, the quantifying an amount of myelination in the neuronal cells is assessed by analyzing an expression of contactin associated protein 1 (Caspr1) in the neuronal cells.

According to some embodiments of the invention, the quantifying an amount of myelination in the neuronal cells is effected by analyzing an expression of Neurofilament (NF) in the neuronal cells.

According to some embodiments of the invention, the quantifying an amount of myelination in the neuronal cells is assessed by analyzing a juxtaposition of the neuronal cells and myelinating cells.

According to some embodiments of the invention, the method further comprises *ex-vivo* differentiating embryonic stem cells into the OPCs prior to step (a).

According to some embodiments of the invention, the method further comprises a step of selecting the OPCs according to an expression of a cell surface marker.

The cell surface marker can be CD140 (PDGFR alpha) or O4.

According to some embodiments of the invention, the ex-vivo differentiating is effected by a method comprising:
(a) culturing in a suspension undifferentiated human embryonic stem cells in the presence of retinoic acid and growth factors to generate neurospheres; and
(b) contacting the neurospheres with a growth factor on an adherent substrate.

According to some embodiments of the invention, the method further comprises a step of contacting the neurospheres in a medium being devoid of the growth factor following step (b).

According to some embodiments of the invention, the contacting is effected for 2-3 days.

According to some embodiments of the invention, the undifferentiated human embryonic stem cells are aggregated.

According to some embodiments of the invention, the growth factor is bFGF, EGF or a combination thereof.

According to some embodiments of the invention, the suspension comprises noggin.

According to some embodiments of the invention, the adherent substrate is a matrigel or an extracellular matrix component.

According to some embodiments of the invention, the step (a) is effected for 10-30 days.

Further disclosed but not forming part of the invention is the method that further comprises manufacturing a pharmaceutical composition comprising the agent which is capable of upregulating myelination of neuronal cells.

The method may further comprise manufacturing a pharmaceutical composition comprising the agent which is capable of downregulating myelination of neuronal cells.

The method may further comprise testing the agent which is capable of upregulating maturation or survival of oligodendrocytes *in-vivo.*

The method may further comprise testing the agent which is capable of down-regulating maturation or survival of oligodendrocytes *in-vivo.*

The method may further comprise manufacturing a pharmaceutical composition comprising the agent which is capable of upregulating maturation or survival of oligodendrocytes.

The method may further comprise manufacturing a pharmaceutical composition comprising the agent which is capable of downregulating maturation or survival of oligodendrocytes.

A pharmaceutical composition comprising an agent identified according to the methods of the present invention is also disclosed.

Further aspects and embodiments are disclosed in the accompanying claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

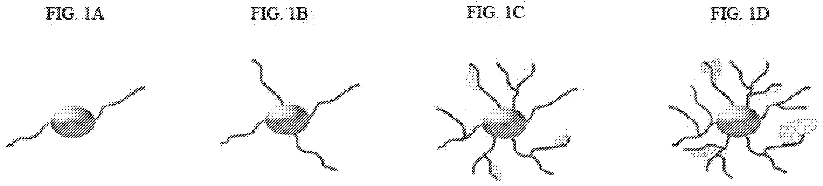

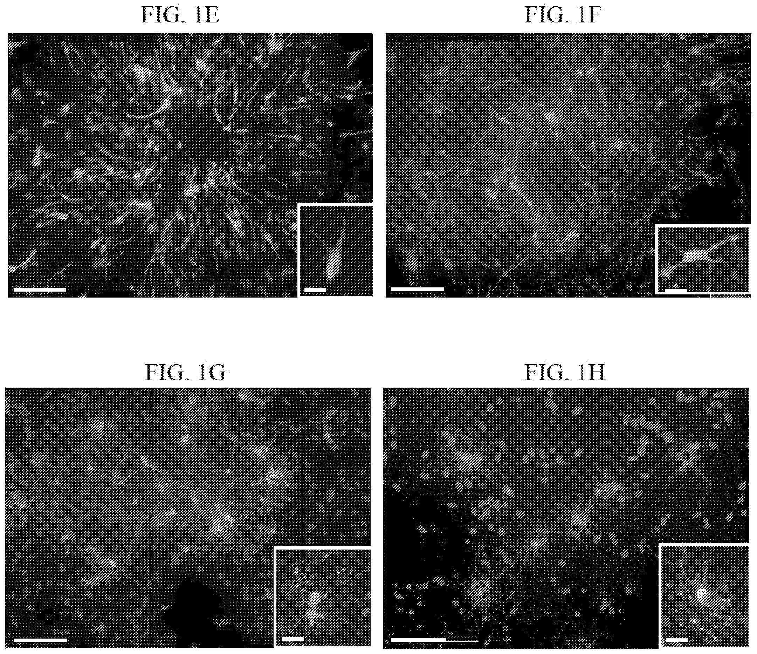

FIGs. 1A-I illustrate that human embryonic stem-OPC (hES-OPC) follow typical oligodendrocytes differentiation *in-vitro.* FIGs. 1A-D: Schematic illustration of OPC differentiation toward mature oligodendrocytes upon growth factors removal; 1A: hES-NSC (neuronal stem cells) grown with growth factors (EGF+bFGF), 1B: hES-OPC grown w/o growth factors for 3 days, 1C: hES-OL (oligodendrocytes) grown without growth factors for 9 days, 1D: hES-OL grown without growth factors for 11 days. FIGs. 1E-H: representative morphology for each differentiation stage described in scheme; 1E:hES-NSC stained with NESTIN (green) and DAPI (blue), 1F: hES-OPC stained with O4 (green) and DAPI (blue), 1G:hES-OL stained with O4 (green) and DAPI (blue), 1H: hES-OL stained with MBP (red) and DAPI (blue). Each differentiation stage contains an inset of enlarged representative cell. 1I: RT-PCR of oligodendrocyte lineage genes, a-d according to schematic illustration in Figures 1A-D. Images scale bar: 100m, insets scale bar: 20m.

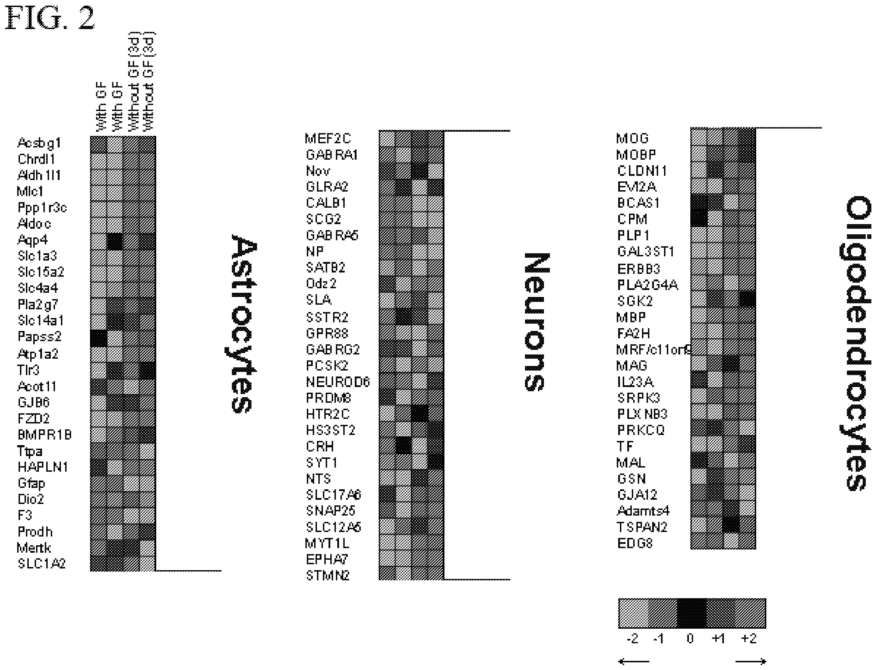

FIG. 2 is a heat map illustrating the clustering of hES-OPC gene profile to CNS specific gene expression. The red squares represent genes which were up regulated during initial (3 days) differentiation, while green squares represent genes which were down regulated during initial (3 days) differentiation. The map is divided into astrocyte, neuron and oligodendrcoyte specific genes which correspond to the thoroughly characterized gene profile of Cahoy et al, 2008, J Neurosci 28, 264-278.

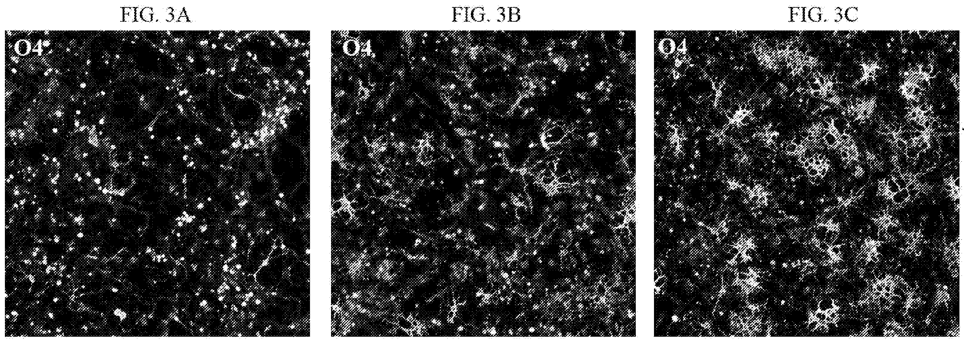

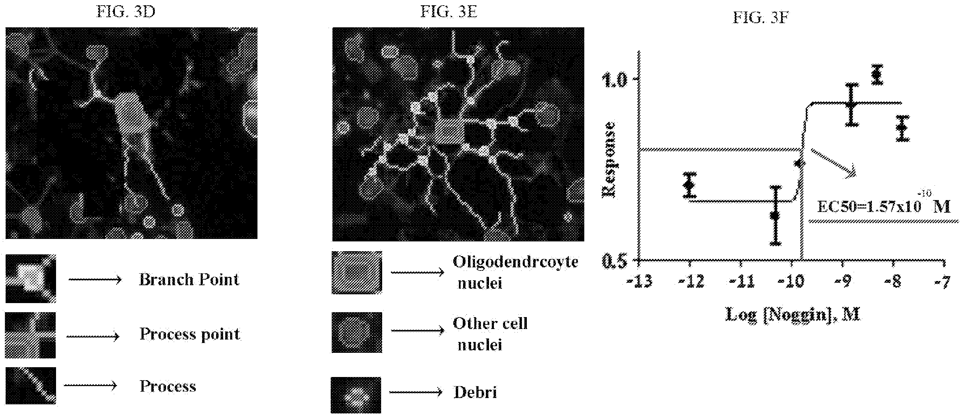

FIGs. 3A-F illustrate a dose response curve of Noggin using differentiation assay. 3A-C: O4 staining of 10 days differentiated hES-OPC, control group (3A), 0.15nM Noggin (3B) and 1.5nM Noggin (3C). 3D-E: Parameters used for analysis of NT (3D) and Noggin (3E) treated cells. 3F: Dose response curve of Noggin which was generated by integrating three parameters: number of O4 positive cells and number and length of oligodendrocyte processes.

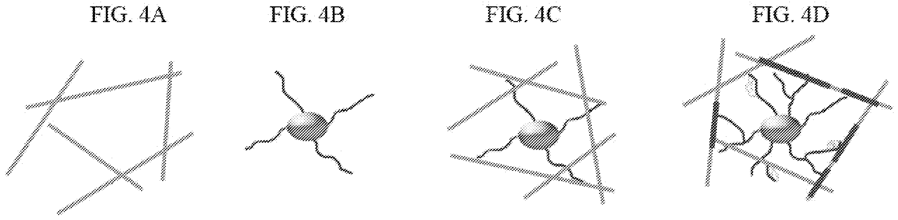

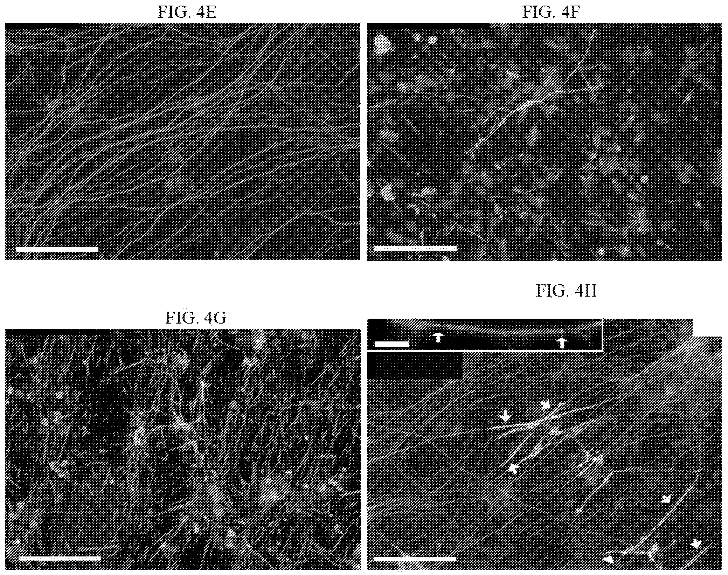

FIGs. 4A-H illustrate that hES derived OPC myelinate DRG neurons *in-vitro.* FIGs. 4A-D: Schematic illustration of co-culture between hES-OPC and DRG neurons. 4A: DRG neurons, 4B: hES-OPC growth without growth factors for three days, 4C: one day co-culture between hES-OPC and DRG neurons. 4D: 18 days co-culture between hES-OPC and DRG neurons. FIGs.4E-H; 4E: DRG neurons network before co-culture (NF staining, red), 4F: hES-OPC grown without growth factors for three days before co-culture (O4 staining, green), 4G: one day co-culture between hES-OPC (O4 staining, green) and DRG neurons (MBP staining, red), 4H: 18 days co-culture between hES-OPC (MBP staining, red) and DRG neurons (NF staining, green), inset: CASPR (green) and MBP (red) staining. Scale bar: 100m, inset scale bar: 20m.

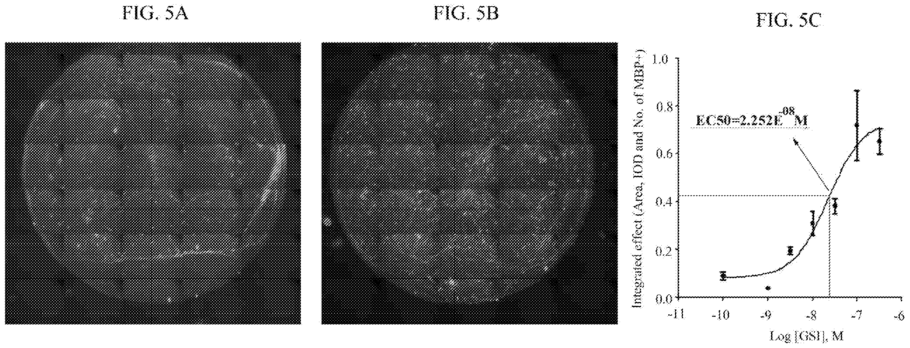

FIGs. 5A-F illustrate a dose response curve of gamma secretase inhibitor X using myelination assay. 5A and 5B: An overview of Neurofilament (green) and MBP (red) staining of a well of 96 well from control (5A) and GSI-X 100nM (5B). 5C. Dose response curve of GSI-X which was generated by integrating three parameters; Area of MBP staining, IOD and number of MBP positive cells. 5D-F: Parameters used for analysis of NT and GSI-X treatment. 5D: MBP staining before identification by the image-pro plus software. 5E: MBP staining identified and analyzed by the image-pro plus software. 5F: Identification of co-localized area between MBP (red) and neurofilament (green) staining. 5D-F scale bar: 100µm.

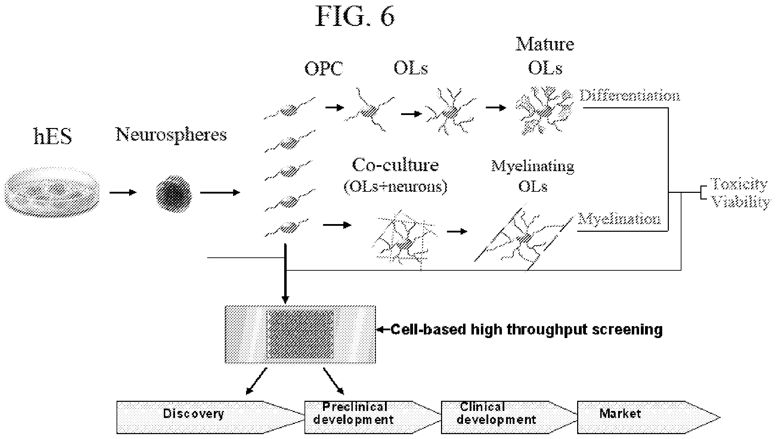

FIG. 6 is a schematic demonstration of the hES-OPC differentiation and myelination assay.

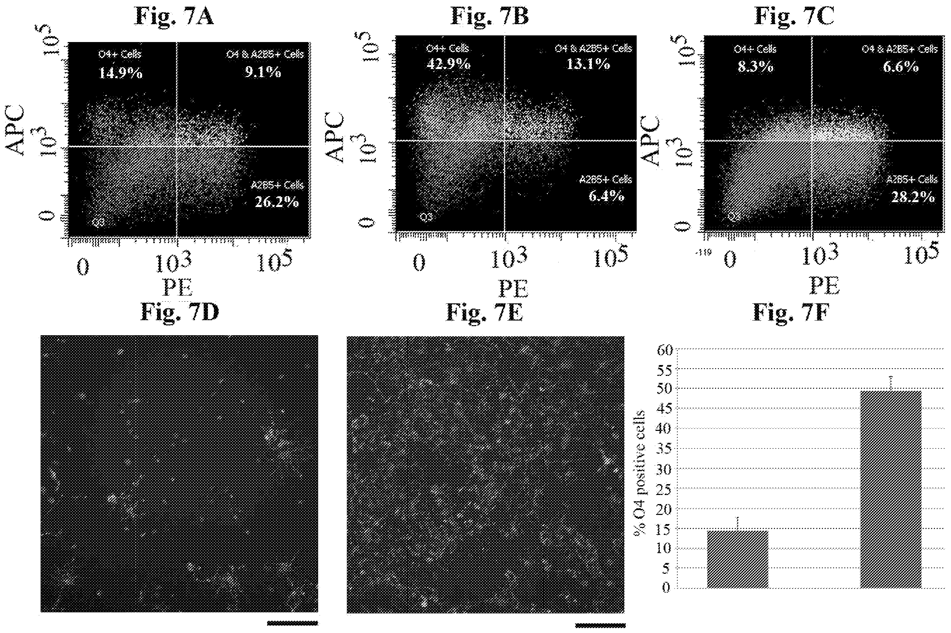

FIGs. 7A-F illustrate the increase in the number of O4+ cells after O4 MACS sorting. FIGs. 7A-C: FACS analysis of O4 (APC- green dots), A2B5 (PE-purple dots) and O4&A2B5 (APC+PE-yellow dots) signaling in Presorted cells (7A), O4 sorted cells (7B) and negative cells (7C). FIGs. 7D-E: O4 (green) and DAPI (blue) staining of presorted (7D) and O4 sorted cells (7E) 48 hours after seeding the cells. 7F: Cellomics analysis of the percentage of O4+ cells before (brown bar), and after (green bar) O4 sorting. Scale bar: 100µm.

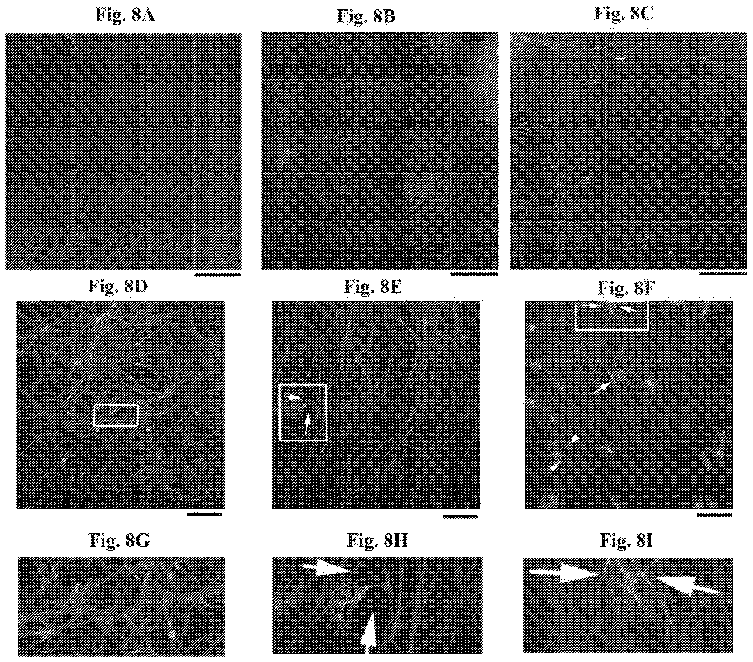

FIGs. 8A-I illustrate the co-culture between DRGs neuron axons and hOPC before and after sorting. Cultures were fixed 9 days after co-culture and stained with NF (green), MBP (red) and DAPI (blue). FIGs. 8A-C: 25 image montages of DRGs without hES-OPC (8A), co-culture between DRGs axons and hES-OPC without O4 sorting (8B) or with O4 sorted hOPC (8C). FIGs. 8D-F: same as FIGs. 8A-C but enlargement of one field only, arrows point myelin fibers. FIGs.8G-I: Enlargement of insets of FIGs. 8D-F respectively, arrows point myelin fibers. Images scale bar: FIGs. 8A-C; 660 µm. FIGs. 8D-F; 100µm.

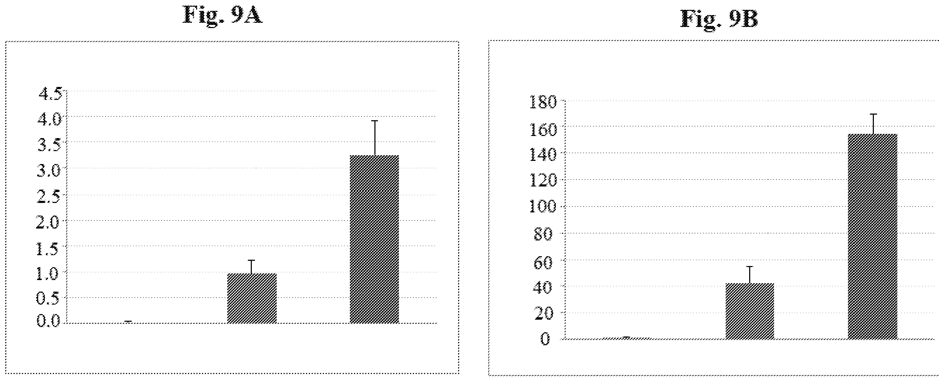

FIGs. 9A-B illustrate the increase in the percentage of MBP+ cells and Myelin fibers in O4 sorted co-culture based on Cellomics Image analysis of 392 fields from 8 wells for each cell treatment. 9A: % MBP+ cells of DRGs only (blue-left bar), pre-sorted cells (Brown- middle bar) and O4 sorted cells (green- right bar). 9B: Same as 9A but instead of % of MBP+ cells the number of myelin fibers (overlap between NF and MBP labeling) were counted.

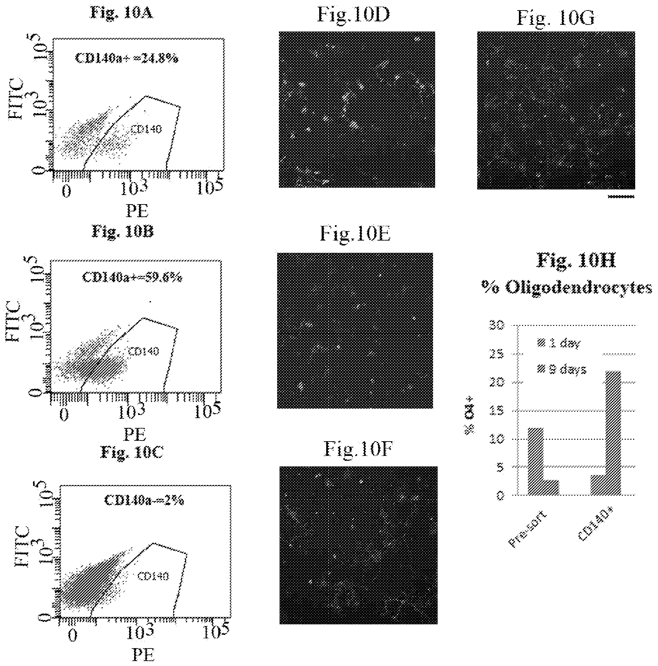

FIGs. 10A-H illustrate the increase in the number of CD140+ cells after CD140+ MACS sorting. Figs. 10A-C show FACS analysis of CD140a (PE- purple dots), in Presorted cells (Fig. 10A), CD140a sorted cells (Fig. 10B) and CD140a- cells (Fig. 10C). Figs. 10D-E: O4 (green), CD140 (red) and DAPI (blue) staining of presorted (Fig.10D) and CD140a sorted cells (Fig.10E) 24 hours after cell seeding post sorting. Figs. 10F-G show O4 labeling nine days after sorting, presorted (Fig.10F) and CD140a sorted cells (Fig. 10G). Fig. 10H shows Cellomics analysis of the percentage of O4+ cells 24hr and 9 days after CD140a sorting. Scale bar: 100µm.

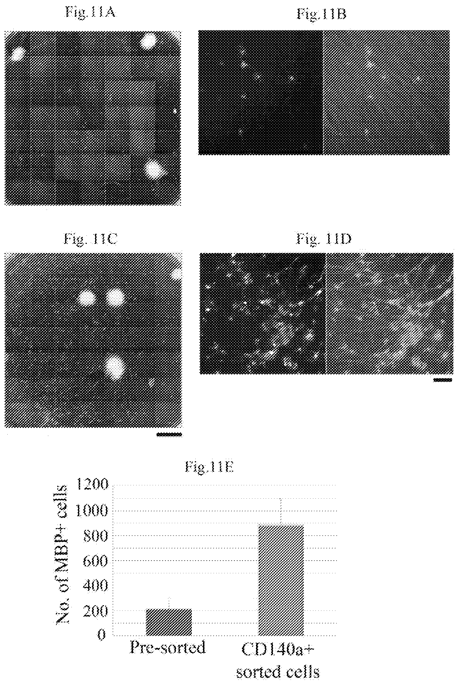

FIGs. 11A-E demonstrate co-culture between DRGs neuron axons and hOPCs before and after CD140a sorting. Cultures were fixed nine days after co-culture and stained with NF (green), MBP (red) and DAPI (blue). Figs. 11A-D show 49 image montages of DRGs with pre-sorted cells (Fig. 11A) or CD140a sorted cells (Fig. 11C), enlargement of one field of each co-culture is presented in figures 11B and 11D respectively. Fig. 11E shows Cellomics analysis of the percentage of MBP+ cells of presorted and CD140a+ cells following nine days of co-culture. Scale bar: Figs. 11A and 11C- 660 µm, and Fig. 11B and 11D- 100µm.

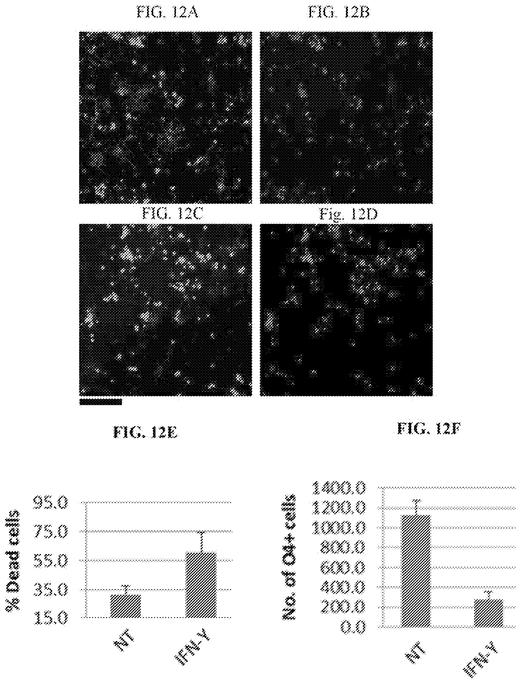

FIGs. 12A-F demonstrate the effect of IFN-Y on survival of human oligodendrocytes. The culture was grown for 14 days. Fig. 12A shows non-treated cells labeled with O4 (green) and DAPI (blue), Fig. 12B shows non-treated cells labeled with dead labeling (red) and DAPI (blue), Fig. 12C shows IFN-Y treated cells labeled with O4 (green) and DAPI (blue), Fig. 12D shows IFN-Y treated cells labeled with dead labeling (red) and DAPI (blue). Figs. 12E-F show statistical analysis of the percentage of dead cells and number of O4+ cells respectively. Scale bar: 100µm.

### DESCRIPTION OF SPECIFIC EMBODIMENTS OF THE INVENTION

The present invention, in some embodiments thereof, relates to a method of identifying agents that affect maturation and survival of oligodendrocytes or myelination of neuronal cells using *ex-vivo* differentiated embryonic stem cells.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Until presently, mechanistic studies analyzing human oligodendrocyte differentiation and functional myelination have been based on rodent assays. A co-culture system using brain derived OPCs from human embryos was recently described. However, the use of human embryos is problematic, due to lack of reproducibility and poor availability.

The invention is based in part on a novel human-specific differentiation and myelination assay which may be used to quantitatively screen for drugs that affect oligodendrocytes (Ols), their precursors, Oligodendrocyte Progenitor Cells (OPCs) and additionally their functionality in interacting with neuronal axons. This assay is based on the use of reproducible cell lines of Ols or OPCs that are *ex-vivo* differentiated from human embryonic stem (ES) cells. The cell lines can be stored in liquid nitrogen and thawed when needed.

The invention enables the use of hES derived OPCs (hES-OPCs) for quantitatively screening agents which up-regulate maturation of OPCs into Ols, their survival and myelination.

Quantifying the effect of one agent (noggin) on a cell line of such hES -OPCs was performed by counting the number of O4 positive cells, the number of processes and branch points (Example 2).

Quantifying the effect of a small molecule agent (a gamma secretase inhibitor) was performed by co-culturing the hES-OPC with dorsal root ganglion (DRG) neuronal axons and counting the number of co-localized of MBP positive fibers and Neurofilament positive fibers as compared to non-treated cells (Examples 3 and 4).

Thus, according to one aspect of the present invention there is provided a method according to claim 1 of quantifying an effect of an agent on myelination of neuronal cells.

According to another aspect, there is provided a method according to claim 2 of quantifying an effect of an agent on maturation, survival or a combination thereof of oligodendrocytes.

As used herein, the term "myelin" refers to the fatty substance which encloses certain axons and nerve fibers, provides essential insulation, and enables the conductivity of nerve cells which transmit electrical messages to and from the brain.

The phrase "myelination of neuronal cells" refers to the ability of oligodendrocytes (i.e. processes extending therefrom) to wrap around neuronal axons and form myelin sheaths.

As used herein, the term "maturation of OPCs" refers to the maturation or differentiation program of OPCs into myelinating oligodendrocytes.

As used herein, the term "survival" is meant any process by which a cell avoids death. The term survival, as used herein, also refers to the prevention of cell loss as evidenced by necrosis, apoptosis, or the prevention of other mechanisms of cell loss. Increasing survival as used herein indicates a decrease in the rate of cell death by at least 10%, 25%, 50%, 75%, 100%, 200% or more relative to an untreated control. The rate of survival may be measured by counting cells capable of being stained with a dye specific for dead cells (e.g., propidium iodide) in culture.

The cells used in the assay of this aspect of the present invention comprise oligodendrocyte precursor cells which have been *ex-vivo* differentiated from embryonic stem cells.

It will be appreciated that the OPCs of this aspect of the present invention have the potential to differentiate into a myelinating phenotype (i.e. to increase myelination *in-vitro*), but are not capable, without further treatment (e.g. in the presence of an agent which induces further differentiation thereof), of myelinating *in-vitro.*

In this aspect of the invention, the OPCs may also have the following functional phenotypes - a mitotic phenotype (i.e. that can divide and be expanded for three or more passages in culture) and migratory capacities.

The OPCs may comprise an elongated, bipolar or multipolar morphology. Typically, the OPCs can incorporate bromodeoxyuridine (BudR), a hallmark of mitosis.

Examples of OPC marker expression include, but are not limited to, PDGF-receptor, O4 sulfatide marker, Nkx2.2, Sox10, Olig1, Olig2, oligodendrocyte specific protein (OSP), 2',3'-cyclic nucleotide-3'-phosphodiesterase (CNP), adenomatous polyposis coli (APC); NG2 (Chondroitin sulfate proteoglycan), A2B5, GD3 (ganglioside), nestin, vimentin and E- or PSA-NCAM.

According to a particular embodiment the OPCs of this aspect of the present invention express Olig1 and Olig2 to a greater extent than they express myelin basic protein (MBP) as measured in an identical assay. Such assays include analysis by Immunohistochemistry, RT-PCR and/or Western blot analysis.

As used herein, the term "to a greater extent" is meant between 2 to 20 fold.

The OPCs of this aspect of the present invention OPCs express each of oligl, olig2 and Hes5.

As mentioned, the OPCs of this aspect of the present invention are *ex-vivo* differentiated from embryonic stem cells (ESCs).

Once human stem cells are obtained, they may be treated to differentiate into OPCs. An exemplary method is described in the Materials and Methods section below. It will be appreciated however, that the present method contemplates additional methods for generating OPCs, which are known in the art.

Thus, as described in Example 1 herein below, cells or aggregates thereof may be subjected to differentiation conditions, which comprise retinoic acids, such that neurospheres are allowed to form. The culture medium used is selected according to the stem cell used. Thus, for example, a medium suitable for ES cell growth, can be, for example, DMEM/F12 (Sigma- Aldrich, St. Lewis, MO) or alpha MEM medium (Life Technologies Inc., Rockville, MD, USA), supplemented with supporting enzymes and hormones. These enzymes can be for example insulin (ActRapid; Novo Nordisk, Bagsværd, DENMARK), progesterone and/or Apo transferrin (Biological Industries, Beit Haemek, Israel). Other ingredients are listed in Example 1 of the Examples section.

As used herein the phrase "retinoic acid" refers to an active form (synthetic or natural) of vitamin A, capable of inducing neural cell differentiation. Examples of retinoic acid forms which can be used in accordance with the invention include, but are not limited to, retinoic acid, retinol, retinal, 11-cis-retinal, all-trans retinoic acid, 13-cis retinoic acid and 9-cis-retinoic acid (all available at Sigma- Aldrich, St. Lewis, MO).

Retinoic acid can be used at a concentration range of 1-50 µM.

The culture medium may be further supplemented with growth factors, which may be present at least in part of the culturing period to promote cell proliferation and facilitate differentiation into the neuronal glial lineages. According to an embodiment of this aspect of the present invention, such growth factors include for example, EGF (5-50 ng/ml) and bFGF (5-50 ng/ml) (R&D Systems, Minneapolis, MN, Biotest, Dreieich, Germany).

As used herein the phrase "neurospheres" refers to quasi-spherical clusters or spheres containing mainly neural stem cells and early multipotent progenitors that can differentiate into neurons, oligodendrocytes and astrocytes as well as other glial cells.

The cells are cultured until ripened neurospheres are formed.

As used herein the phrase "ripened neurospheres" refers to neurospheres in which some of the neural stem cells have differentiated to become specialized oligodendrocyte progenitors having acquired makers of the oligodendrocyte lineage (e.g. Sox10, Nkx2.2., NG2, A2B5), while others have differentiated to become neural progenitors or astrocytes progenitors.

According to an embodiment of the invention the cells are allowed to culture, for example for 10-30 (e.g., 20-30) days, at the end of which detached neurospheres are formed. The spheres, or cells dissociated therefrom, are then adhered to adherent substrates (e.g. matrigel or an extracellular matrix component (e.g., collagen, laminin and fibronectin) and subjected to further expansion with growth factors and eventually to differentiation after removal of growth factors on a cationic adherent substrate (e.g. poly-D- lysine or Polyornithine with fibronectin (FN).

In order to avoid generation of terminally differentiated myelinating cells, the OPCs are not left at this stage in the absence of growth factors for more than three, four or five days.

Following neuroglial sphere formation (or concomitantly with) or following culture on adherent substrate, the cells may be incubated with an agent capable of inhibiting Bone Morphogenetic Protein (BMP) activity.

As used herein the term "agent capable of down-regulating BMP activity" refers to an agent which can at least partially reduce the function (i.e., activity and/or expression) of BMP.

BMP reducing agents include cystine knot-containing BMP antagonists, which are divided into three subfamilies, based on the size of the cystine ring; CAN (eight-membered ring), twisted gastrulation (nine-membered ring), and chordin and noggin (10-membered ring). The CAN family is divided further based on a conserved arrangement of additional cysteine residues, and includes gremlin and PRDC; cerberus and coco; DAN; USAG-1 and sclerostin.

Other BMP inhibitors include, but are not limited to, chordin like BMP inhibitor (CHL2), Neuralin (also homologous to chordin) which behave as secreted BMP-binding inhibitors; inhibin (belongs to the TGF-β superfamily); follistatin (which binds to inhibin); GDF3, an inhibitor of its own subfamily (TGF-β), which blocks classic BMP signaling in multiple contexts; Crossveinless-2 (hCV-2), a BMP function inhibitor; Ectodin (available at Qiagene, Valencia, CA), which is homologous to sclerostin and inhibits the activity of BMP2, BMP4, BMP6, and BMP7; connective tissue growth factor (CTGF), a BMP receptor antagonist; BMP-3, a BMP receptor antagonist; and gp130 signaling cytokines.

It will be appreciated that oligonucleotide inhibitors, which down-regulate expression of BMP genes (e.g., siRNA) may also be used in accordance with this aspect of the present invention. Methods of genetically modifying stem cells are well known in the art.

The agent which can be used to inhibit BMP can be noggin (e.g., 10-100 ng/ml, e.g., 50 ng/ml used as a Noggin-Fc chimera available from R&D systems, Minneapolis, NM). As mentioned, noggin can be used in any culturing step following, and optionally with retinoic acid treatment.

The time and period of treatment with the BMP inhibitor following neurosphere formation affects the differentiation level of the cells. The amount and timing of noggin culture may be adjusted so as to generate OPCs at a particular differentiation stage.

Noggin may also be added during the ripening of Neuro Spheres (NS).

The cells used for the assay of the present invention may be freshly generated or frozen cells. Typically, if the cells are frozen they are thawed in medium comprising growth factors (e.g. bFGF and EGF). The cells are typically cultured for at least one day following thawing in the absence of growth factors prior to the start of the assay.

Agents which may be tested using the assay of the invention include small molecule agents, chemicals, peptides, proteins, polynucleotide agents (e.g. siRNA agents). It will be appreciated that agents which are already known to affect myelination may also be used in this assay in order to quantify their optimum dose.

Contacting the cells with the agent can be performed by for example adding the agent to the cells such that the agent is in direct contact with the cells. The conditions used for incubating the cells are selected for a time period/concentration of cells/concentration of agent/ratio between cells and agent and the like, which enable the agent to induce cellular changes, such as changes in transcription and/or translation rate of specific genes, which are analyzed. The cells can be contacted with the agent for at least 3 days, 5 days, 7 days, 10 days, 14 days or at least 21 days.

Quantifying the effect of agents refers to the capability to grade the ability of the agent to affect myelination using a numerical value as opposed to determine whether the agent is simply able to affect myelination or not.

The quantification may be determined either by analyzing the number of cells which show the myelinating phenotype or by quantifying the amount of myelination on a particular number of cells (e.g. by determining the level of expression of a myelinating marker).

In order to perform the assay in a quantitative way, care should be taken when seeding the cells, such that the same number of cells is added to each assay dish and well. A 96 well plate can be used to culture the OPCs. An exemplary number of OPCs to seed in one well of a 96 well plate is at least 5000. Each dish or well of a 96 well plate can be coated with Matrigel and PDL.

Quantitative assessment of an agent may be effected by analyzing an expression level of a marker of myelination - such markers included myelin basic protein (MBP), myelin oligodendrocyte glycoprotein (MOG), myelin associated glycoprotein (MAG), galactocerebroside and/or proteolipid protein (PLP). Typically, an increase in expression of any of these markers in the human oligodendrocyte is indicative of an agent which increases (either an increase in the total number of cells which express and/or an increase in the level of expression) the myelination potential of the OPCs.

Quantitative measurement of expression levels of the above identified markers (and/or additional markers as further described herein below) may be effected using methods known in the art including, RT-PCR analysis, Western Blot analysis and immunohistochemistry.

Another method of quantifying a marker of myelination is by counting the number and/or measuring length of the OPC processes and/or counting the number of branch points in the OPCs. This may be performed using an antibody which recognizes OPCs such as an antibody which recognizes O4, 2'-5' Cyclic Nucleotide 3' phosphodiesterase (CNPase), as well as transcription factors oligl and olig 2.

Immunohistochemistry methods can be used such that additional cell parameters as well as expression of a myelinating marker may be studied.

Typically, the method requires the use of an antibody, which specifically recognizes the marker, wherein the antibody includes a detectable marker. Such markers include but are not limited to fluorescent moieties, peroxidase moieties, biotin moieties and phosphorescent moieties. Examples of antibodies which may be used in the context of the present assay are provided in the Examples section herein below. Anti-galactocerebroside antibodies are commercially available, for example, from Sigma.

In order to quantitatively analyze the effect of an agent on the myelinating potential of the OPCs, typically the percentage of cells in a particular field which express the marker of myelination is estimated. In some cases, only cells which co-express both a marker of myelination and a nuclear marker are counted (e.g. DAPI).

Many methods are known in the art to count the total number of cells per well or per field including but not limited to DAPI staining or Hoecht staining.

The number of O4/MBP positive processes can be counted and measured only if attached to O4/MBP cell body. The lengths of the OPC processes can be counted. Alternatively, or additionally, the number of branch points per O4 or MBP positive cell body can be counted.

As well as (or instead of) analyzing markers of myelination in the OPCs of the invention, the present inventors also contemplate adding neuronal cells to the assay system so that the functional activity (i.e. myelinating potential) of the OPCs may be measured directly.

Any neuronal cells are contemplated by the invention as long as they are capable of being myelinated by oligodendrocytes.

Mouse dorsal root ganglion cells can be used. Such cells may be dissociated from mouse embryo (e.g. embryonic day 13 (E13)) and seeded on plates. The neuronal cells may be grown in the appropriate medium (e.g. Neurobasal (NB) medium).

The neuronal cells can be treated so as to eliminate Schwann cells and fibroblasts from the culture e.g. treatment with 10µM uridine/10µM 5'-Fluoro 2'-deoxyuridine (Sigma) to eliminate fibroblasts and Schwann cells.

Once sufficient neuronal cells are obtained, OPCs (preferably those which have been deprived of growth factors for 1-3 days) may be seeded thereupon. NB/N2B27 medium (1v/1v) supplemented with VitC (e.g. 50µg/ml) may be used for the co-culture.

Following seeding, (e.g. 12-24 hours later), the agent may be added to the culture and after an appropriate amount of time (e.g. 4-45 days), the culture is analyzed.

The number of neuronal cells which are wrapped by myelin can be counted (e.g. by counting the number of neuronal cells which are ensheathed by MBP positive or O4 positive cells which form myelin fibers).

Cells staining positively for proteins known to be adjacent to the nodes may be visualized, such as contactin associated protein, Caspr1 (contactin-associated protein 1) may also be counted. Expression of such proteins suggests that the *in-vitro* myelination reproduces the structure of myelin internodes and paranodes, since the condensation of Caspr at the paranode indicates a functional organization of node of Ranvier, necessary for rapid current conduction along the axon - see for example, Example 3 herein below.

Quantifying the amount of myelination in the neuronal cells may also be performed by analyzing a juxtaposition of the neuronal cells and myelinating cells.

Thus, neuronal cells staining positively for neurofilament proteins may be counted only when it is evident that they are ensheathed in MBP or O4 positive cells - see for example, Example 4 herein below.

It will be appreciated that computer programs may be used to integrate the data obtained from the immunohistochemistry analysis. Such programs are commercially available or may be produced in house by those skilled in the art.

The present inventors have found that enrichment of myelin producing cells may be performed by selecting for CD140a (PDGFRA) positive hESC derived OPC or O4 positive hESC derived OPC.

Typically, the selecting is effected using antibodies that are capable of specifically recognizing these cell-surface proteins, although the present invention contemplates additional agents such as polynucleotides or small molecules.

The enriching may be effected using known cell sorting procedures such as by using a fluorescence-activated cell sorter (FACS).

As used herein, the term "flow cytometry" refers to an assay in which the proportion of a material (e.g. OPC comprising a particular maker) in a sample is determined by labeling the material (e.g., by binding a labeled antibody to the material), causing a fluid stream containing the material to pass through a beam of light, separating the light emitted from the sample into constituent wavelengths by a series of filters and mirrors, and detecting the light.

A multitude of flow cytometers are commercially available including for e.g. Becton Dickinson FACScan and FACScalibur (BD Biosciences, Mountain View, CA). Antibodies that may be used for FACS analysis are taught in Schlossman S, Boumell L, et al, [Leucocyte Typing V. New York: Oxford University Press; 1995] and are widely commercially available.

If the CD140a or O4 antibody is attached to a magnetic moiety (either directly, or indirectly through a cognate binding molecule), the heterogeneous cell population may be enriched for cells by magnetic activated cell separation.

If the CD140a or O4 antibody is attached to an affinity moiety, the heterogeneous cell population may be enriched for CD140a or O4 ⁺ cells by affinity purification with the cognate binding molecule. Thus, for example, if the CD140a or O4 antibody is attached to biotin, the heterogenous cell population may be depleted of CD140a or O4⁺ by purification with Streptavidin beads or column. The CD140a or O4⁺ cells can subsequently be retrieved. If, for example the CD140a or O4⁺ antibody is attached to an antibody or an Fc of an antibody, the heterogeneous cell population may be depleted of CD140a or O4⁺ by purification with protein A beads or column. The CD140a or O4⁺ cells can subsequently be retrieved.

It will be appreciated that since the differentiated cells of this aspect of the present invention typically grow as adherent clusters, prior to cell sorting the heterogenous cell population should be dispersed using a dispersing agent.

Examples of dispersing agents include, but are not limited to dispase, collagenase, accutase and trypsin. Alternatively, or additionally trituration may also be performed to increase the dispersal of the cells.

Once a novel candidate agent shows that it is capable of up-regulating the myelinating potential of OPCs, their maturation or survival in the presently described assay, its optimal dose may be tested using the same *in-vitro* tests. The candidate agent's myelinating potential may also be tested in animal models for diseases associated with reduced myelination such as animal models for Multiple Sclerosis (such as mice treated with antigens to become Experimental Autoimmune Encephalitis (EAE) mice).

Once its pro-myelination, maturation or survival potential has been corroborated, the agent may be synthesized in commercial amounts and/or pharmaceutical compositions comprising same may be synthesized, as described herein below.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the agent which upregulates myelination, maturation or survival accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

The agents identified using the assay of the present invention may be used in the *ex-vivo* generation of myelinating cells from stem cells. Alternatively, the agents may be administered directly to a patient suffering from a disease associated with lack of myelination. Such diseases include, but are not limited to multiple sclerosis, acute disseminated encephalomyelitis, transverse myelitis, chronic inflammatory demyelinating polyneuropathy, Guillain-Barré Syndrome, central pontine myelinosis, inherited demyelinating diseases such as Leukodystrophy, and Charcot Marie Tooth.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader..

### MATERIALS AND METHODS

### Generation of cells used for the assay

Human ES cell lines were cultured at 37 °C in 5 % CO₂ on feeder layers of human new-born foreskin fibroblasts (HEF). The growth medium (ES1) consisted of DMEM/F12 (Sigma), 14 % knockout serum replacement (KSR), nonessential amino acids (1/100), 100 mM beta-mercaptoethanol (all three from Invitrogen/Gibco), 1 mM Na pyruvate, 2 µg/ml heparin (Sigma) and 8 ng/ml human recombinant basic FGF (FGF-2; Preprotech). Five days after seeding, hES cell colonies were detached with 1.2 mg/ml collagenase IV (Worthington) for 45-90 minutes at 37 °C. The aggregates were further cultured as above, or subjected to differentiation as described (Izrael et al Mol Cell Neurosci 34, 310-323 (2007)).

***Step 1-transition:*** For this transition step, hES cell colonies were transferred to tissue culture plates in medium T (Transition) consisting of 50 % (v/v) ES1 medium and 50 % (v/v) of ITTSPP/B27medium. ITTSPP/B27 is itself a mixture of one volume of DMEM/F12 containing 2 % B27 supplement (Invitrogen) and one volume of DMEM/F12 containing 25 µg/ml human insulin (ActRapid, Novo Nordisk), 50 µg/ml human Apo-transferrin (Biological Industries, Israel), 6.3 ng/ml progesterone, 10 µg/ml putrescine, 50 ng/ml sodium selenite and 40 ng/ml triiodothyronine (T3) (all from Sigma), with 100 U/ml penicillin and 100 µg/ml streptomycin. Medium T was supplemented by 20 ng/ml r-human EGF (R&D Systems) and 4 ng/ml r-human bFGF. After 1 day, the bulk of non-adherent hES cells colonies and aggregates were transferred to 6 cm bacterial (non-adherent) plates and grown in T medium supplemented with 20 ng/ml r-human EGF (R&D Systems) and 2 ng/ml r-human bFGF.

***Step 2: All-trans retinoic acid (ATRA) treatment:*** The medium was switched to ITTSPP/B27, with 20 ng/ml EGF and 10 mM ATRA additions. Medium was changed daily for 7 days.

***Step 3: Suspension culture:*** During this step, which allows for ripening of neurospheres (NS), the culture was continued in ITTSPP/B27 medium with 20 ng/ml EGF for 18 days. Medium was changed every other day.

### Neural stem cells proliferation and differentiation

***Step 4: Matrigel adhesion 1:*** Spheres/clusters were transferred to 6-well tissue culture plates that had been coated for 1.5 h at room temperature (RT) with BD Matrigel (growth factor reduced Matrigel, BD Biosciences) diluted 1:30 in DMEM/F12. The culture medium was ITTSPP/B27 with 20 ng/ml EGF and after one day, any non-adherent aggregates were discarded. Medium was changed every other day for 7-9 days.

***Step 5: Matrigel adhesion 2:*** The neurospheres/clusters which were free from fibroblast and epithelial cells were selected and picked on a new Matrigel plate. Neurospheres (not more than 10 NS) were detached and partially dissociated with 0.025% trypsin (InVitrogen) in PBS (Ca+ and Mg2+ free), for 2-3 min at 37 °C (passage 1). The trypsin was neutralized by one volume of trypsin inhibitor (Invitrogen) and four volumes of 2 mg/ml BSA in ITTSPP/B27. The cells were spun at 1200 rpm for five minutes in an Eppendorf centrifuge. The dissociated small clusters were re-seeded onto Matrigel-coated plates (at 1:1 to 1:3 ratio) in ITTSPP/B27 with 20 ng/ml EGF (step 4). After 2-3 weeks, the cells were dissociated by trypsin treatment as above (passage 2) and the resulting hES -NS cells were seeded on plates coated with poly-D-lysine (PDL, 100 µg/ml) and mouse laminin (20 µg/ml) or PDL (20 µg/ml) and fibronectin 5 µg/ml in ITTSPP/B27 with 20 ng/ml EGF, for one day. At each new seeding, 1 µg/ml mouse laminin (Sigma) was added to the medium.

***Steps 6 and 7: Expand on poly-D-lysine:*** After one day, the medium was changed to N2/B27 medium consisting of DMEM/F12 with 0.5% (v/v) N2 supplement (Invitrogen), 1% (v/v) B27 supplement. Growth factors EGF and bFGF were added at10 ng/ml each. After 7-14 days, the cells from PDL plates were split (1:2 or 1:3, passage 2 (P2)) by trypsinization to obtain monolayers. After one week, cells were collected with trypsin and frozen in 0.5x10⁶ cells ampoule (P3). Further passages were conducted weekly, when indicated.

***Step 8: GF removal:*** At different passages, the terminal differentiation was initiated by removing the growth factors. Whenever growth factors were removed, 1 µg/ml mouse laminin, 50 ng/ml noggin and 50 µg/ml vitamin C were added to the N2/B27 medium. Cells were thawed in N2/B27 medium supplemented with growth factors bFGF and EGF. One day before splitting and seeding in 96 well plates, growth factors were removed.

### Differentiation assay; dose response of Noggin

5000 hES-OPC were seeded in each well of Matrigel and PDL-coated 96 well black clear plates (CI-3603, Corning). The medium used for the experiment comprised DMEM/F12 with 0.5% (v/v) N2 supplement (Invitrogen), 1% (v/v) B27 supplement, Glutamax and 100 U/ml penicillin and 100 µg/ml streptomycin (Biological Industries, IL). The cells were deprived from growth factors 24 hours prior to seeding. 12 hours following seeding, increasing doses of Noggin (Preprotech, Israel) were supplemented to the media for the ten days of differentiation. The Noggin doses ranged between 15 nM to 0.015 nM (450 ng/ml to 0.45 ng/ml) with 3.16 fold dilutions (square root of 10) in the presence of 0.1 % DMSO and 50 µg/ml Vitamin C. Samples without Noggin in the presence of 0.1% DMSO and 50 µg/ml Vitamin C served as negative control. The media was changed every other day by replenishing 2/3 of the media. On day ten, the wells were fixed using 4 % paraformaldehyde (PFA) for further O4 and Dapi staining.

### Co-culture between DRG neurons and hES-OPC

***Mouse DRG neuron purification:*** DRG dissociated cells from mouse embryonic day 13 (E13) were seeded on matrigel and PDL-coated 12 well plates (30,000 cells/well) or seeded on 96 well black clear plates (CI-3603, Getter) (7000 cell/well) and grown in NB medium for two weeks prior hES-OPC addition. NB media consist of Neurobasal medium (Gibco) supplemented with B27 (Gibco) and 50 ng/ml NGF (Alomone labs). At the first week of DRG culture, the cells were treated with two cycles (two days each) of NB medium containing 10 µM uridine/10 µM 5'-Fluoro 2'-deoxyuridine (Sigma) to eliminate fibroblasts and Schwann cells.

***Co-culture with hES-OPC:*** Following two weeks of DRG neuron culture, 50,000 (on 12 well plate) or 10,000 (on 96 well plate) hES derived-OPC that were grown in the absence of GF for 3 days were seeded on DRG neurons. The co-culture was conducted in NB/N2B27 medium (1v/1v) supplemented with VitC 50µg/ml, NGF was omitted from DRG neurons culture medium, two days prior hES-OPC addition. Co-cultures were fixed and RNA extraction and immunohistochemistry (IHC) were conducted at different time points in order to evaluate differentiation and myelination.

***Myelination assay-dose response of gamma secretase inhibitor X:*** hES-OPCs which were deprived from growth factors (EGF and bFGF) for one day were seeded on top of DRGs neurons. 12 hours after seeding, increasing doses of gamma secretase inhibitor X (GSI-X, MBS565771, Calbiochem) were supplemented to the media for the 21 days of differentiation. GSI-X doses ranged between 1 nM to 1 µM with 3.16 folds (square root of 10) in the presence of 0.1 % DMSO and 50 µg/ml Vitamin C. Wells without GSI with 0.1% DMSO and 50µg/ml Vitamin C served as a control. The media was changed every other day by replenishing 2/3 of the media. On day 21 the wells were fixed using 4 % PFA for further MBP, Neurofilament-160kd and Dapi staining (detailed below).

### Gene expression analysis

***RNA and gene expression analysis:*** Total RNA was isolated using RNeasy (Qiagen) and cDNA transcribed with reverse transcriptase RT M-MLV (Promega) or Superscript II (Invitrogen). The PCR reactions were conducted with Super Therm Taq polymerase. The primers sequences of oligodeoxynucleotide for each of the indicated genes are provided in Table 1, herein below. The PCR conditions were as followed; amplification: 94 °C, 2 minutes then 30 cycles (except 22 cycles for GAPDH) at 94 °C, 30 seconds; 52-58 °C, 30 seconds; 72 °C, 1 minute. The amplified DNA bands were photographed under UV-light, scanned, and their intensity was quantified using the AlphaEase spot density software. Band intensity was verified to be in the linear range by varying the amount of PCR reaction loaded on the gels.

**Table 1**

| ***Accession number*** | ***Sequence*** | ***Human Gene*** |
|---|---|---|
| NM_002509 | TGCCTCTCCTTCTGAACCTTGG SEQ ID NO: 1 | Nkx2.2 F |
| | GCGAAATCTGCCACCAGTTG SEQ ID NO: 2 | Nkx2.2 R |
| NM_002046 | CATGTAGGCCATGAGGTCCACCAC SEQ ID NO: 3 | GAPDH F |
| | TGAAGGTCGGTGTGAACGGATTTGGC SEQ ID NO: 4 | GAPDH R |
| NM_002055 | TGGCCAAGCCAGACCTCAC SEQ ID NO: 5 | GFAP F |
| | CATCTCCACGGTCTTCACCAC SEQ ID NO: 6 | GFAP R |
| NM_001010926 | CAGTTCCTGACGCTCCAC SEQ ID NO: 7 | HES5 F |
| | CGGCACTACAAATATCATAGAAC SEQ ID NO: 8 | HES5 R |
| NM_138983 | TTGCATCCAGTGTTCCCGATTTAC SEQ ID NO: 9 | OLIG1 F |
| | TGCCAGTTAAATTCGGCTACTACC SEQ ID NO: 10 | OLIG1 R |
| NM_005806 | AAGGAGGCAGTGGCTTCAAGTC SEQ ID NO: 11 | OLIG2 F |
| | CGCTCACCAGTCGCTTCATC SEQ ID NO: 12 | OLIG2 R |
| NM_000533 | CTGCTCACCTTCATGATTGC SEQ ID NO: 13 | PLP F |
| | TGACTTGCAGTTGGGAAGTC SEQ ID NO: 14 | PLP R |
| NM_001001995 | GTATGTCATCTATGGAACTGC SEQ ID NO: 15 | PLP/DM20 F |
| | GTAATGTACACAGGCACAGC SEQ ID NO: 16 | PLP/DM20 R |
| NM_006941 | GCCTGTTCTCCTGGGGCTTTGCTGC SEQ ID NO: 17 | SOX 10 F |
| | CATCCACCTCACAGATCGCCTACAC SEQ ID NO: 18 | SOX 10 R |
| NM_001025081 | CCATGGCTAGACGCTGAAAAC SEQ ID NO: 19 | MBP F |
| | GAAGGAACGTGATCTTCACAC SEQ ID NO: 20 | MBP R |

***Microarrays:*** Total RNA was extracted using the RNneasy micro kit (Qiagen, Valencia, CA). The quality of total RNA was assessed using RNA 6000 Nano LabChip (Agilent Technologies, CA, USA) and Bio-analyzer microchip (Agilent Technologies, CA, and USA). Two independent biological replicates of RNA extracts for each experimental condition were used to prepared cDNA and biotin-labeled cRNA for hybridization. DNA microarray analyses were carried out with Affymetrix Human Gene 1.0 ST array according to the Affymetrix standard protocol (Affymetrix, Santa Clara, CA). Fold induction analysis performed with MAS 5 statistical algorithms at the Crown Human Genome Center, Weizmann Institute of Science.

***Immunostaining:*** Cells were fixed using 4 % paraformaldehyde (PFA), washed with PBS and kept at 4 °C before staining. Non-specific staining was blocked with normal goat serum (5 % w/v in PBS) for 30 minutes at RT. Primary mouse monoclonal (mMc) and rabbit polyclonal antibodies, diluted in 1 % goat serum, were as described in Table 2, herein below. All staining was followed by the nuclear fluorescent dye DAPI (Sigma, 0.05 µg/ml). All coverslips were mounted in Mowiol (Calbiochem, La Jolla, CA). An Olympus IX-70 FLA microscope with a DVC-1310C digital camera (DVC, Austin, TX) was used and images processed with Photoshop and analyzed with Image-Pro-Plus software (Media Cybernetics, Silver Spring, MD) to measure Integrated Optic Density (IOD) of color-specific pixels and MBP positive fiber length. HSC Scan-Array VTI (Cellomics, Thermo-dynamics) was used for scanning and processing the images of O4 positive cells by using the neural profiling module. Statistical analyses by two-tailed Student *t*-test and ANOVA were with Instat (GraphPad Software Inc., San Diego, CA) or JMP 5.0 software (SAS Institute, Cary, NC).

**Table 2**

| Purchased at | Dilution | ***Primary Ab*** |
|---|---|---|
| R&D Systems | 1:1000 | Mouse IgM monoclonal anti-O4 |
| Covance | 1:1000 * | Mouse IgG1 monoclonal anti-tubulin-βIII |
| Abcam Inc | 1:1000 * | Mouse monoclonal Neurofilament-M (ab7794) |
| Abcam Inc | 1:750 * | Rabbit polyclonal Neurofilament (ab9034) |
| Abcam Inc | 1:100 * | Rat anti Myelin basic protein (MBP) |
| Santa Cruz Bio | 1:250* | Rabbit polyclonal to CASPR (sc-25669) |
| Abcam Inc | 1:500 | Rabbit polyclonal Nestin antibody (ab5968). |

| | | ***Secondary Antibodies*** |
|---|---|---|
| Chemicon | 1:75 | Goat anti-mouse IgM-FITC |
| Jackson Lab | 1:500 | Goat anti-rat IgG Cy3-conjugated Affipure |
| Jackson Lab | 1:500 | Goat anti-rabbit Cy3-conjugated |
| Jackson Lab | 1:500 | Goat anti rat IgG Cy5-conjugated Affipure |
| Molecular probes | 1:500 | Goat anti mouse IgM-Alexa fluor red 594 |
| Molecular probes | 1:500 | Goat anti mouse IgG-Alexa flour green 488 |
| Abcam Inc | 1:400 | Goat anti-rat IgG -FITC |

***Magnetic activated cell sorting (MACS) with O4 microbeads:*** 10⁷ hOPCs were incubated with conjugated anti-O4 microbeads (Miltenyi Biotec, 130-094-543, 10µl) for 15 minutes at 4°C, according to the manufacturer's protocol. The cells were washed by adding 2 ml of phosphate-buffered saline (PBS), PH 7.2, and 0.5% bovine serum albumin (BSA) and centrifuged at 300xg for ten minutes. The supernatant was aspirated completely and up to 10⁷ cells were re-suspended in 500µl of buffer.

The magnetic separation was conducted using MS column and miniMACS separator (Miltenyi Biotec,) according to the manufacturer's protocol.

***Magnetic activated cell sorting (MACS) with CD140a*+*:*** 10⁷ hOPCs were incubated with Human Anti Alpha PDGFR-PE-100T (BD, PMG556002, 10µl) for 15 minutes at 4°C according to the manufacturer's protocol. The cells were washed by adding 2 ml of phosphate-buffered saline (PBS), PH 7.2, and 0.5% bovine serum albumin (BSA) and centrifuged at 300xg for ten minutes. The supernatant was aspirated completely and up to 10⁷ cells were re-suspended in 80µl of buffer.

Anti-PE microbeads (Miltenyi Biotec, 130-048-801, 20µl) were added to 10⁷ cells and incubated for 15 minutes at 4°C. The cells were washed by adding 2 ml of phosphate-buffered saline (PBS), PH 7.2, and 0.5% bovine serum albumin (BSA) and centrifuged at 300xg for 10 minutes. The supernatant was aspirated completely and up to 10⁸ cells were re-suspended in 500µl of buffer.

The magnetic separation was conducted using LS column and miniMACS separator (Miltenyi Biotec,) according to the manufacturer's protocol.

***FACS analysis of pre-sorted cells, sorted cells and the negative cell population:*** 1x10⁵ (100µl) of pre-sorted cells, sorted cells and the negative cell population were transferred to Eppendorf tubes for FACS analysis. For each sample (10-20µl) of the primary antibody were added (Anti-O4-APC, Miltenyi Biotec, 130-095-891) and incubated at 4°C for 30 minutes. The cells were washed by adding FACS buffer (Ca2+ and Mg2+-free PBS with 0.2-2% BSA or HSA) to bring cell suspension to 1.3 ml. The cells were centrifuge at 1680 RPM (300 g) for five minutes at 4°C, re-suspended in 200µl FACS buffer, passed into FACS tubes through their mesh and analyzed on the flow cytometer (BD Biosciences, Mountain View, CA) according to the manufacturer's protocol.

### EXAMPLE 1

### Generation and characterization of cells used in the myelination assay

The human ES cells were differentiated by a stepwise protocol, where neural spheres develop and grow in suspension with EGF. Neural spheres cells were then separated from non-neural tissues by attachment to Matrigel extracellular matrix and growth with EGF. To investigate the effect of drugs on oligodendrocyte function, it is important to use well-characterized pools of human oligodendrocyte progenitor cells that may be differentiated by a simple procedure *in-vitro* or *in-vivo.* Proliferation of neural sphere cells, dissociated with trypsin, on Poly D-Lysine-coated plates for two to three passages in the presence of growth factors EGF and bFGF, yields lines of neural stem cells (NSC) available for further differentiation studies. Each line results from the dissociation of pools of neural spheres.

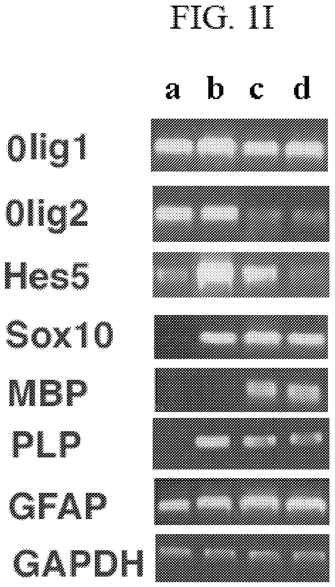

*In-vitro* and *in-vivo,* CNS OLs develop from NSC through a series of morphological steps, as summarized in Figures 1A-D, and corresponding to the expression of specific markers. In NSC pools, OPCs derived from human ES cells progress through different stages upon growth factor withdrawal (Figures 1E-I). The ES derived cells treated with growth factors contain thin bipolar neural progenitors expressing the intermediate filament Nestin, characterizing rodent and human NSCs (Figures 1A and 1E). These cells already express the transcription factors oligl and olig2, characterizing the oligodendrocyte lineage and motor neuron progenitors (Figure 1I). Upon growth factor removal, cells acquire a specific sulfatide at their surface, which is recognized by the O4 monoclonal antibody. Three days after the start of differentiation, O4 positive cells have very few processes (Figure 1B and 1F). In the same cells, the mRNA of SOX10, a transcription factor controlling positively the myelination process was detected as well as the mRNA of the proteolipid protein PLP, a component of myelin (Figure 1I). Later, around ten to eleven days after growth factor withdrawal, cells have thicker cytoplasmic processes, and express MBP, the myelin basic protein, responsible for tight wrapping of myelin sheaths around axons (Figures 1D, 1H and 1I).

Two NSC lines were characterized more thoroughly by analysis of gene expression levels on human cDNA microarrays (Affymetrix). cDNA was derived from duplicate samples of NSCs, grown in the presence or absence of growth factors for three days. Genes expressed in the cells were compared with sets of genes that do not overlap one with the other and neurons, astrocytes and oligodendrocytes were characterized. The results show that NSCs proliferating in the presence of growth factors (left lanes) express only 4-5 out of 27 genes characterizing either astrocytes or oligodendrocytes. After growth factor removal, cells differentiate and express 24 out of 27 genes characterizing astrocytes or oligodendrocytes (Figure 2). In contrast, neuronal-specific genes do not segregate with either undifferentiated or differentiated status of the hES derived cells (Figure 2), indicating that the NSCs lines at passage 6 used herein contain glial progenitors, rather than neuronal progenitors.

### EXAMPLE 2

### Differentiation of NSCs into multibranched OLs; quantification of the effect of Noggin

BMP signaling negatively regulates oligodendrocyte lineage development, and it has been shown that Noggin, an inhibitor of BMP signaling, promotes the differentiation of ES-derived oligodendrocytes. As a proof of concept that effects of drugs on oligodendrocyte differentiation can be quantified, the inventors searched for the optimal concentration of Noggin to be used to promote differentiation. NSCs at fifth passage were distributed to wells of 96 well dishes and submitted to differentiation in the absence or the presence of Noggin in serial dilutions 1:3.16 at concentrations varying from150 ng/ml to 0.5 ng/ml (4.7x10⁻⁹ to 1.5x10⁻¹¹M) in triplicate assays. After ten days without growth factors, cells were stained with O4 monoclonal antibody, and nuclei counterstained with DAPI. Noggin added after growth factor removal stimulates the emergence of OLs with longer and multiple processes versus bipolar O4 positive cells in the absence of Noggin (Figures 3A-C). High throughput microscopy was followed by analysis with the neuronal profiling bio application associated with the same apparatus (Array-scan VTI HSC, Cellomics, Thermofisher). Figures 3D-E demonstrate examples of the quantification of different parameters of OL morphology. Nuclear DNA staining with DAPI was used to quantify the cell number per field, and oligodendrocytes were characterized by staining with O4. Small and dense DAPI stained granules (representing nuclear fragments or apoptotic cells, red circles in Figures 3D-E) were rejected, as well as O4-positively stained cells not associated with DAPI. Similarly, O4 positive processes (green) were counted and measured only if attached to O4 cell body. Places where one process becomes two were counted as branch point (Figures 3D-E, yellow circles). The numbers of processes, their lengths, and the number of branch points per O4 positive cell body were counted. Figures 3D-E show specific examples of O4 positive cell morphologies and corresponding measurements obtained with the application. It was found that the process numbers per cell, the total process length per well, and number of branch points per cell correlated with Noggin concentration. Thus, it may be concluded that the present assay is capable of quantifying the effect of Noggin. An EC50 curve was generated by integrating the parameters above, 1.57*10⁻¹⁰ M of Noggin was found to generate 50 % of the maximal effect (Figure 3F). However the number of O4 positive cell per well was not changed with noggin treatment. This result demonstrates that hES derived OPCs can be used in-vitro to screen for drugs or chemicals modulating OL differentiation.

### EXAMPLE 3

### Co-culture of hES derived OPCs and mouse dorsal root ganglia neurons

To address mechanistic aspects of myelination in the CNS, a system of co-cultures of purified neurons and oligodendrocytes was used.

Since the hES derived OPC can differentiate to mature oligodendrocytes, the inventors asked whether these cells can myelinate neurons *in-vitro* or *in-vivo.*

As targets of myelination, mouse embryonic dorsal root ganglia (DRG) neurons were used. The DRG neurons were obtained from mouse embryonic day13 (E13) and seeded on Matrigel and PDL-coated plates. The neurons were cultured for two weeks in the presence of NGF, and during the first week two cycles of FuDR treatment eliminated Schwann cells from the culture. Pictures of the neurons, hOPC and co-culture of both are shown in Figures 4E-H and schematized in Figures 4A-D. After two weeks a network of Neurofilament positive neurons axons was formed (Figure 4E) and no MBP positive cells were observed on or in between the DRG neuron culture. O4 positive hOPC were deprived of GF for 3 days and seeded on top of the axon network.

Figures 4A-D demonstrate the different types of cultures, while the cells after immune-staining are represented in Figures 4E-H: the DRG neurons only, the NSC three days after growth factor removal and the co-culture for 24 hours or for 18 days. While shortly after being in contact with neurons, OPCs acquire a branched morphology, after 18 days of contact, the mature OL express MBP and wrap the axons to form myelin fibers. In the newly formed myelinating sheath internodes, proteins known to be adjacent to the nodes were visualized, such as contactin associated protein, Caspr1 (contactin-associated protein 1). In Figure 4H inset, Caspr positive staining (green) was observed at the edges of the myelin fibers (arrows). This suggests that the in-vitro myelination reproduces the structure of myelin internodes and paranodes, since the condensation of Caspr at the paranode indicates a functional organization of node of Ranvier, necessary for rapid current conduction along the axon.

### EXAMPLE 4

### A dose response study on the effect of gamma secretase inhibitor X on the formation of MBP positive cells

Triplicate of the co-culture experiments were treated with serial dilution of another gamma secretase inhibitor (gamma secretase inhibitor X, Calbiochem) in order to determine the effect of this inhibitor on oligodendrocyte differentiation.

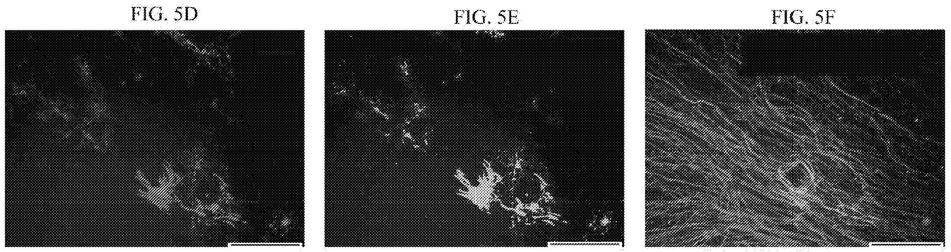

After 21 days, the neurons were stained with anti neurofilament Ab (Figure 5A and B, green) and the oligodendrocytes with anti MBP antibodies (Figure 5A and B, red). Juxtaposition of automatic pictures recorded with an Olympus microscope linked to the cell selector AVISO reflects that the inhibitor increases MBP staining (Figures 5A and B). Analysis of the integrated and normalized value of the area, the integrated optical density (IOD), and number of the MBP positive staining was used for generating a dose response curve (Figure 5C). The log of the dose of Gamma secretase inhibitor reveals a typical dose response curve with half maximal effect (EC50), concentration of 2.25x10-8M. Figures 5D-E, demonstrate the automatic recognition of MBP signal (Figure 5D - before recognition and Figure 5E - after recognition), and analysis of Neurofilament and MBP fibers superimposition (Figure 5F).

### EXAMPLE 5

### Enrichment of O4 positive hESC derived OPC by MACS sorting

In order to improve the yield of OPC and immature oligodendrocytes population in the hES-OPC culture, the cells were sorted with O4 microbead antibody. The sorting was conducted on hOPC that were deprived from growth factors (bFGF and EGF) and supplemented with Noggin. O4 expression was then detected by IHC and FACS techniques. Figures 7A-C show the levels of O4+ cells, O4+ and A2B5+ double positive cells and A2B5+ cells for three cell conditions; cells before sorting (pre-sorted cells), O4 sorted cells and the negative cells. The percentage of O4+ cells was increased from 24% to 56% (2.3 fold increase) as demonstrated in Figures 7A- B. In other O4 sorting experiments O4+ cells population were enriched to 90% (not shown). The enrichment that was established was further demonstrated by staining with O4 antibody 48 hours after the cells were seeded (Figures 7D-E). The percentage of both FACS and immunohistochemistry (O4 and Dapi staining) are similar as can be seen by comparing image analysis of % O4+ cells (Figure 7F, conducted by using Cellomics) and FACS analysis (Figures 7A-C, green positive dots in the first quarter).

In order to test whether myelination can be improved by O4 sorting, the myelination capacity of hESC-OPC was compared with and without O4 sorting. As shown in Figures 8, no MBP+ cells were presented in the negative control group (Figures 8A, 8D and 8G- DRGs only). When hESC-OPC cells were added to the DRGs, MBP+ cells can be found distributed between DRGs axons with few myelin fibers after nine days of co-culture (Figures 8B, 8E and 8H). However, an increase in the number of MBP+ cells and myelination were found when O4 sorted hESC-OPC were co-cultured with DRGs neurons axons (Figures 8C, 8F and 8I). A 3-4 fold increase was measured (using Cellomics) in the % MBP+ and the number of Myelin fibers (number of overlap areas between NF and MBP staining) in the O4 sorted co-culture (Figures 9A-B).

### EXAMPLE 6

### Enrichment of CD140a (PDGFRA) positive hESC derived OPC by MACS sorting

In order to improve the yield of OPC progenitor population in the hES-OPC culture, the cells were sorted with oligodendrocytes progenitor early marker CD140a (PDGFRa). This was conducted on hOPC that were deprived from growth factors (bFGF and EGF) and supplemented with Noggin. CD140a expression was then detected by IHC and FACS techniques. Figures 10A-H demonstrate the levels of CD140+ cells in each cell population; cells before sorting (pre-sorted cells), CD140a+ sorted cells and CD140a- cells. The percentage of CD140a+ cells was increased from 24.8% to 59.6% (2.4 fold increase) as demonstrated in Figures 10A-B. Most of CD140a+ sorted culture (above 70%) was CD140a positive while the number of O4+ cells decreased when compared to pre-sorted culture (Figures 10D-E). This demonstrates that early OPC progenitor cells with proliferation and migration potential can be sorted by CD140a antibody. Next, it was demonstrated that these early progenitor can differentiate into mature oligodendrocytes by staining the cells with O4 antibody after nine days of culture. Figure 10H shows an increase in the percentage of O4 positive cells in the CD140a+ sorted cells versus to presorted fraction. The depletion of O4+ cell population from CD140a+ population resulted in greater enrichment of early OPC subpopulation (not shown).

Next, it was tested whether CD140a+ sorting improve the potential of cells to become myelinating MBP+ cells. A comparison between myelination capacity of hESC-OPC with and without CD140 sorting was conducted. For that aim presorted cells or CD140a+ sorted cells were co-culture with DRGs neuron axons. Figures 11A-D, show that very few MBP+ cells were presented in pre-sorted co-culture (Figures. 11A-B), while more MBP+ cells developed from CD140a+ cells (Figures 11C-D). The number of myelin fibers (overlapping area between NF and MBP) was increase in CD140a sorted population as illustrated in Figure 11D. Four fold in the number of MBP+ cells was measured in CD140a+ co-culture versus to pre-sorted co-culture within nine days (Figure 11E).

### EXAMPLE 7

### Human Oligodendrocytes and hESC-OPC survival assay

In order to evaluate the survival of human oligodendrocytes or its progenitor cells in culture, a specific dead cell labeling (Live/dead viability/cytotoxicity kit for mammalian cells, Invitrogen, molecular probes, L3223) was used. A culture of differentiated oligodendrocytes and oligodendrocytes progenitor culture sorted by O4+ or CD140+ MACS sorting respectively were obtained. The effect of recombinant human IFN-Y (R&D systems, Biotest, 285-IF-100) was tested on oligodendrocytes survival. Following 14 days in culture the cells were labeled with dead cell labeling (Ethidium heterodimer 1-red labeling) and O4 labeling (green). It was found that IFN-Y increased the percentage of dead cells and decreased the number of differentiated oligodendrocytes (Figures 12A-F). Such an assay gives complementary results on the effect of tested compound on oligodendrocytes survival.

## Claims

1. A method of quantifying an effect of an agent on myelination of neuronal cells, the method comprising:
(a) ex-vivo differentiating embryonic stem cells into oligodendrocyte precursor cells (OPCs) expressing Hes5, oligl and olig2;
(b) freezing and thawing said OPCs, wherein thawing is performed in a medium comprising growth factors;
(c) after thawing, culturing said OPCs for at least one day in the absence of growth factors;
(d) contacting said OPCs with the agent; and
(e) quantifying at least one marker of myelination in said OPCs, thereby quantifying the effect of the agent on myelination of neuronal cells.

2. A method of quantifying an effect of an agent on maturation, survival or a combination thereof of oligodendrocytes, the method comprising:
(a) ex-vivo differentiating embryonic stem cells into oligodendrocyte precursor cells (OPCs) expressing Hes5, oligl and olig2;
(b) freezing and thawing said OPCs, wherein thawing is performed in a medium comprising growth factors;
(c) after thawing, culturing said OPCs for at least one day in the absence of growth factors;
(d) contacting said OPCs with the agent; and
(e) quantifying at least one marker of maturation, survival or a combination thereof in the oligodendrocytes, thereby quantifying the effect of the agent on maturation, survival or a combination thereof of said oligodendrocytes

3. The method of claims 1 or 2,
wherein said OPCs express oligl and olig2 to a greater extent than they express myelin basic protein (MBP); or
wherein said marker is a polypeptide selected from the group consisting of myelin basic protein (MBP), myelin oligodendrocyte glycoprotein (MOG), galactocerebroside (GalC), myelin associated glycoprotein (MAG), 2'3'-cyclic nucleotide-30-phosphodiesterase (CNP), O4 and proteo lipid protein (PLP); or
wherein said quantifying is conducted by counting the number of O4 stained cells with at least two processes, the percentage of O4 positive cells, the number of processes per O4 stained cells, the total length of the processes per O4 stained cells, the number of branch points per O4 stained cells, the number of MBP positive cells, calculating the percentage of MBP positive cells, or measuring the area of myelin membranes per stained cells or the area of the O4 stained processes; or
wherein said agent is a small molecule.

4. The method of claims 1 or 2, wherein said OPCs are present in a culture comprising neuronal cells.

5. The method of claim 4, wherein said culture is devoid of Schwann cells.

6. The method of claim 4, further comprising quantifying an amount of myelination of neuronal cells in said culture comprising neuronal cells.

7. The method of claim 6,
wherein said quantifying an amount of myelination in said neuronal cells is effected by analyzing an expression of contactin associated protein, (Caspr1) in said neuronal cells; or
wherein said quantifying an amount of myelination in said neuronal cells is effected by analyzing an expression of Neurofilament (NF) in said neuronal cells; or
wherein said quantifying an amount of myelination in said neuronal cells is effected by analyzing a juxtaposition of said neuronal cells and myelinating cells.

8. The method of claim 1, wherein said ex-vivo differentiating is effected by a method comprising:
(a) culturing in a suspension undifferentiated human embryonic stem cells in the presence of retinoic acid and growth factors to generate neurospheres; and
(b) contacting said neurospheres with a growth factor on an adherent substrate.

9. The method of claim 8, further comprising a step of contacting said neurospheres in a medium being devoid of said growth factor following step (b).

10. The method of claim 9,
wherein said contacting is effected for 2-3 days; or
wherein said medium being devoid of growth factors comprises noggin.

11. The method of claim 8,
wherein the undifferentiated human embryonic stem cells are aggregated; or
wherein said growth factor is bFGF, EGF or a combination thereof; or
wherein the suspension comprises noggin; or
wherein said adherent substrate is a matrigel or an extracellular matrix component; or wherein step (a) is effected for 10-30 days.

## Patentansprüche

1. Verfahren zur Quantifizierung des Effekts eines Myelinisierungs-Mittels auf die Myelinisierung neuronaler Zellen, wobei das Verfahren umfasst:
(a) ex-vivo Differenzieren embryonaler Stamm-Zellen zu Oligodendrozyten-Vorläuferzellen (OPCs), welche Hes5, olig1 und olig2 exprimieren;
(b) Einfrieren und Auftauen der OPCs, wobei das Auftauen in einem Medium durchgeführt wird, welches Wachstumsfaktoren umfasst;
(c) nach dem Auftauen, Züchten der OPCs für mindestens einen Tag in der Abwesenheit von Wachstumsfaktoren;
(d) Inkontaktbringen der OPCs mit dem Mittel; und
(e) Quantifizieren mindestens eines Myelinisierungs-Markers in den OPCs, wodurch der Effekt des Mittels auf die Myelinisierung neuronaler Zellen quantifiziert wird.

2. Verfahren zur Quantifizierung eines Effekts eines Mittels auf die Reifung, das Überleben oder eine Kombination davon von Oligodendrozyten, wobei das Verfahren umfasst:
(a) ex-vivo Differenzieren embryonaler Stammzellen in Oligodentrocyten-Vorläuferzellen (OPCs), welche Hes5, olig1 und olig2 exprimieren;
(b) Einfrieren und Auftauen der OPCs, wobei das Auftauen in einem Medium durchgeführt wird, welches Wachstumsfaktoren umfasst;
(c) nach dem Auftauen, Züchten der OPCs für mindestens einen Tag in der Abwesenheit von Wachstumsfaktoren;
(d) Inkontaktbringen der OPCs mit dem Mittel; und
(e) Quantifizieren mindestens eines Markers der Reifung, des Überlebens oder einer Kombination davon in den Oligodendrozyten, wodurch der Effekt des Mittels auf die Reifung, das Überleben oder eine Kombination davon der Oligodendrozyten quantifiziert wird.

3. Verfahren nach Ansprüchen 1 oder 2,
worin die OPCs olig1 und olig2 in einem größeren Ausmaß exprimieren als sie das Mylein-Grundprotein (MBP) exprimieren; oder
worin der Marker ein Polypeptid ist ausgewählt aus der Gruppe bestehend aus Myelin-Grundprotein (MBP), Myelin-Oligodendrozyt-Glycoprotein (MOG), Galactocerebrosid (GalC), Myelin assoziiertes Glycoprotein (MAG), 2'3'-zyklisches Nucleotid-30-phosphodiesterase (CNP), O4 und Proteolipidprotein (PLP); oder
worin das Quantifizieren durchgeführt wird durch Zählen der Anzahl O4 gefärbter Zellen mit mindestens zwei Verfahren, den Prozentsatz O4 positiver Zellen, die Anzahl an Verfahren pro O4 gefärbter Zellen, die Gesamtlänge der Verfahren pro O4 gefärbter Zellen, die Anzahl an Verzweigungspunkten pro O4 gefärbter Zellen, die Anzahl an MBP positiven Zellen, Berechnen des Prozentsatzes an MBP positiven Zellen, oder Erfassen des Bereichs an Myelinmembranen pro gefärbter Zellen oder den Bereich O4 gefärbter Verfahren; oder
worin das Mittel ein kleines Molekül ist.

4. Verfahren nach Ansprüchen 1 oder 2, worin die OPCs in einer Kultur vorhanden sind, welche neuronale Zellen umfasst.

5. Verfahren nach Anspruch 4, worin die Kultur keine Schwann-Zellen aufweist.

6. Verfahren nach Anspruch 4, welches weiter umfasst, Quantifizieren eines Myelinisierungs-Grads neuronaler Zellen in der Kultur, welche neuronale Zellen umfasst.

7. Verfahren nach Anspruch 6,
worin das Quantifizieren eines Myelinisierungs-Grads in den neuronalen Zellen bewirkt wird durch Analysieren der Expression von Contactin assoziiertem Protein, (Caspr1) in den neuronalen Zellen; oder
worin das Quantifizieren eines Myelinisierungs-Grads in den neuronale Zellen bewirkt wird durch Analysieren der Expression von Neurofilament (NF) in den neuronalen Zellen; oder
worin das Quantifizieren eines Myelinisierungs-Grads in den neuronale Zellen bewirkt wird durch Analysieren einer Juxtaposition neuronaler Zellen und myelinisierender Zellen.

8. Verfahren nach Anspruch 1, worin das ex-vivo Differenzieren bewirkt wird durch ein Verfahren, welches umfasst:
(a) Züchten undifferenzierter humaner embryonaler Stammzellen in einer Suspension in der Anwesenheit von Retinsäure und Wachstumsfaktoren, um Neurosphären zu erzeugen; und
(b) Inkontaktbringen der Neurosphären mit einem Wachstumsfaktor an einem haftenden Substrat.

9. Verfahren nach Anspruch 8, welches weiter den Schritt umfasst, Inkontaktbringen der Neurosphären in einem Medium, das keinen Wachstumsfaktor nach Schritt (b) enthält.

10. Verfahren nach Anspruch 9,
worin das Inkontaktbringen für 2-3 Tage durchgeführt wird; oder
worin das Medium, das keine Wachstumsfaktoren enthält, Noggin umfasst.

11. Verfahren nach Anspruch 8,
worin die undifferenzierten humanen embryonalen Stammzellen aggregiert sind; oder
worin der Wachstumsfaktor bFGF, EGF oder eine Kombination davon ist; oder
worin die Suspension Noggin umfasst; oder
worin das haftende Substrat ein Matrigel ist oder eine extrazelluläre Matrixkomponente; oder
worin Schritt (a) für 10-30 Tage durchgeführt wird.

## Revendications

1. Procédé de quantification d'un effet d'un agent sur la myélinisation de cellules neuronales, le procédé comprenant :
(a) la différenciation ex vivo de cellules souches embryonnaires en cellules précurseurs d'oligodendrocytes (OPC) exprimant Hes5, olig1 et olig2 ;
(b) la congélation et la décongélation desdites OPC, la décongélation étant effectuée dans un milieu comprenant des facteurs de croissance ;
(c) après décongélation, la culture desdites OPC pendant au moins un jour en l'absence de facteurs de croissance ;
(d) la mise en contact desdites OPC avec l'agent ; et
(e) la quantification d'au moins un marqueur de myélinisation dans lesdites OPC, ce qui permet de quantifier l'effet de l'agent sur la myélinisation de cellules neuronales.

2. Procédé de quantification d'un effet d'un agent sur la maturation, la survie ou une combinaison de celles-ci d'oligodendrocytes, le procédé comprenant :
(a) la différenciation ex vivo de cellules souches embryonnaires en cellules précurseurs d'oligodendrocytes (OPC) exprimant Hes5, olig1 et olig2 ;
(b) la congélation et la décongélation desdites OPC, la décongélation étant effectuée dans un milieu comprenant des facteurs de croissance ;
(c) après décongélation, la culture desdites OPC pendant au moins un jour en l'absence de facteurs de croissance ;
(d) la mise en contact desdites OPC avec l'agent ; et
(e) la quantification d'au moins un marqueur de maturation, de survie ou une combinaison de celles-ci dans les oligodendrocytes, ce qui permet de quantifier l'effet de l'agent sur la maturation, la survie ou une combinaison de celles-ci desdits oligodendrocytes.

3. Procédé selon les revendications 1 ou 2,
dans lequel lesdites OPC expriment olig1 et olig2 dans une plus grande mesure qu'elles n'expriment la protéine basique de la myéline (MBP) ; ou
dans lequel ledit marqueur est un polypeptide choisi dans le groupe constitué par la protéine basique de la myéline (MBP), la glycoprotéine de myéline oligodendrocytaire (MOG), le galactocérébroside (GalC), la glycoprotéine associée à la myéline (MAG), la 2'3'-nucléotide cyclique-30-phosphodiestérase (CNP), O4 et la protéine protéolipide (PLP) ; ou
dans lequel ladite quantification est conduite en comptant le nombre de cellules colorées par O4 avec au moins deux procédés, le pourcentage de cellules positives à O4, le nombre de procédés par cellules colorées par O4, la longueur totale des procédés par cellules colorées par O4, le nombre de points de ramification par cellules colorées par O4, le nombre de cellules positives à la MBP, le calcul du pourcentage de cellules positives à la MBP, ou en mesurant la surface de membranes de myéline par cellules colorées ou la surface des procédés colorés par O4 ; ou
dans lequel ledit agent est une petite molécule.

4. Procédé selon les revendications 1 ou 2, dans lequel lesdites OPC sont présentes dans une culture comprenant des cellules neuronales.

5. Procédé selon la revendication 4, dans lequel ladite culture est dépourvue de cellules de Schwann.

6. Procédé selon la revendication 4, comprenant en outre la quantification d'une quantité de myélinisation de cellules neuronales dans ladite culture comprenant des cellules neuronales.

7. Procédé selon la revendication 6,
dans lequel ladite quantification d'une quantité de myélinisation dans lesdites cellules neuronales est effectuée par analyse d'une expression de protéine associée à la contactine. (Caspr1) dans lesdites cellules neuronales ; ou
dans lequel ladite quantification d'une quantité de myélinisation dans lesdites cellules neuronales est effectuée par analyse d'une expression de Neurofilament (NF) dans lesdites cellules neuronales ; ou
dans lequel ladite quantification d'une quantité de myélinisation dans lesdites cellules neuronales est effectuée par analyse d'une juxtaposition desdites cellules neuronales et desdites cellules de myélinisation.

8. Procédé selon la revendication 1, dans lequel ladite différenciation ex vivo est effectuée par un procédé comprenant :
(a) la culture dans une suspension de cellules souches embryonnaires humaines non différenciées en présence d'acide rétinoïque et de facteurs de croissance pour générer des neurosphères ; et
(b) la mise en contact desdites neurosphères avec un facteur de croissance sur un substrat adhérent.

9. Procédé selon la revendication 8, comprenant en outre une étape de mise en contact desdites neurosphères dans un milieu dépourvu dudit facteur de croissance après l'étape (b).

10. Procédé selon la revendication 9,
dans lequel ladite mise en contact est effectuée pendant 2 à 3 jours ; ou
dans lequel ledit milieu dépourvu de facteurs de croissance comprend de la noggine.

11. Procédé selon la revendication 8,
dans lequel les cellules souches embryonnaires humaines non différenciées sont agrégées ; ou
dans lequel ledit facteur de croissance est le bFGF, l'EGF ou une combinaison de ceux-ci ; ou
dans lequel la suspension comprend de la noggine ; ou
dans lequel ledit substrat adhérent est un matrigel ou un composant de matrice extracellulaire ; ou
dans lequel l'étape (a) est effectuée pendant 10 à 30 jours.
